# EUROPEAN PATENT SPECIFICATION

(11) **EP 4 604 893 B1**
(45) Date of publication and mention of the grant of the patent: **31.12.2025**
(21) Application number: 24738036.3
(22) Date of filing: 21.06.2024
(51) Int. Cl.: A61F 13/02, A61F 13/00

(54) **WOUND DRESSING**
WUNDVERBAND
PANSEMENT

(30) Priority: 23.06.2023 US 202363509810 P; 03.08.2023 GB 202311951
(43) Date of publication of application: 27.08.2025
(60) Divisional application: 25216556.8
(73) Proprietor: ConvaTec Limited, Deeside, Flintshire CH5 2NU (GB)
(72) Inventor: DAVIES, Liam, Deeside Flintshire CH5 2NU (GB); BROWN, Natalie, Deeside Flintshire CH5 2NU (GB); EADE, Annchalee, Deeside Flintshire CH5 2NU (GB); GASKELL, Beth, Deeside Flintshire CH5 2NU (GB)
(74) Representative: Wilson Gunn
(86) International application number: PCT/GB2024/051590
(87) International publication number: WO 2024/261492

(56) References cited:
- CN-A- 113 543 757
- US-A1- 2017 049 627
- US-A1- 2018 140 755
- US-A1- 2020 170 843

## Description

### Technical Field of the Invention

The present invention relates to the shape of a wound dressing, in particular to the shape of a wound dressing for use with a source of non-atmospheric pressure.

### Background to the Invention

It is known to make wound dressings for use on wounds to aid healing and prevent infections. Such dressings may be required to adhere to a patient's body in whichever location the wound is present. This may require a dressing to be adhered to areas which are variably or highly contoured or which experience a relatively high level of movement. This can make the initial adherence of the dressing challenging and limit subsequent wear time.

To address this problem, practitioners will often use additional securing means to attach a dressing which results in a additional time, cost and reduced comfort for the patient. To help reduce this, manufacturers efforts have often focussed on providing dressings with site specific shapes. However, this not only complicates design and manufacturing of the different shape types and sizes of the dressings, but also requires health care providers to stock many different types and sizes of dressings. Further, it requires practitioners to be educated and/or experienced in many different types of wound dressing to reduce the risk of the dressings being used incorrectly.

Furthermore, where the wound dressing is used with a source of non-atmospheric pressure, for example in Negative Pressure Wound Therapy, it is important to ensure that teh wound dressing provides a sufficient degree of sealing over the wound to allow the pressure differential to be applied consistently.

US2017/049627A1 describes a negative pressure wound therapy system, including an ester-based material adapted to be directly applied to the wound, such as a smooth muscle fistula, without substantially damaging tissue in the wound during dressing changes. The ester-based material may have an affinity for the wound bed surface and/or wound fluid.

CN113543757A describes a wound treatment device having electronic components integrated into a wound dressing. The wound dressing device comprising a wound dressing. The wound dressing including an absorbent material, an electronics unit including a negative pressure source integrated into the wound dressing and at least partially encapsulated by a preformed film material or film. The preformed film material being incorporated into the wound dressing and including apertures configured to allow fluid communication between the absorbent material and the source of negative pressure.

US2018/140755A1 describes an apparatus and method for aspirating, irrigating and/or cleansing wounds. The apparatus and method include one or more of the following: simultaneous aspiration and irrigation of the wound, supplying of thermal energy to fluid circulated through the wound; supplying physiologically active agents to the wound; a biodegradable scaffold in contact with the wound bed; and application of stress or flow stress to the wound bed.

US2020/170843A1 describes sealing compositions for negative pressure treatment systems and wound dressing systems, for sealing a trimmable dressing, having a main dressing portion or cell in fluid (e.g., gas) communication with additional dressing portions or cells, for use in the treatment of wounds. In particular, some embodiments are directed to compositions for improving the versatility of wound dressings for wounds of different shapes or sizes.

The present invention seeks to provide a wound dressing having a shape which helps alleviate some of the issues of existing dressing shapes.

### Summary of the Invention

The present invention provides a wound dressing according to the appended claims.

The first and second elongate portions may be arranged at an angle of between 0 and 180° to one another, preferably between 30 and 150° to one another, more preferably between 45 and 135° to one another, for example between 60 and 120° to one another, such as between 75 and 105° to one another, e.g. about 90° to one another, i.e. the first and second elongate portions may be arranged perpendicular to one another.

The wound dressing may comprise two wings. A first wing may extend from a first long edge of the first elongate portion to the long edge of the second elongate portion. A second wing may extend from a second long edge of the first elongate portion to the long edge of the second elongate portion.

The or each wing may have a triangular shape. The or each wing may have an edge with a curved profile. The or each wing may have an edge with a concave arc profile.

The concave arc may have a radius of curvature of at least 20 mm, at least, 25 mm, at least 30 mm, at least 35mm, at least 40 mm, at least 45 mm, at least 50 mm, at least 55 mm, at least 60 mm, at least 65 mm, at least 70 mm, at least 75 mm or at least 80 mm.

The concave arc may have a radius of curvature of between 20 and 80 mm, preferably between 20 and 600 mm, more preferably between 25 and 45mm, for example, between 28 and 32mm, such as 30 mm.

Advantageously, the concave arc having such a radius of curvature provides a sufficient degree of improve conformity on contours of the wound site and increase in absorbent pad volume proximate to the wound site without hindering the ability of the dressing to be folded abruptly (for example around a heel) or unduly increasing the bulk of the dressing.

The concave arc may have a radius of curvature of at least 20% the width of one or both elongate portions. The concave arc may have a radius of curvature of at least 25% the width of one or both elongate portions. The concave arc may have a radius of curvature of at least 30% the width of one or both elongate portions. The concave arc may have a radius of curvature of at least 35% the width of one or both elongate portions. The concave arc may have a radius of curvature of at least 40% the width of one or both elongate portions. The concave arc may have a radius of curvature of at least 45% the width of one or both elongate portions. The concave arc may have a radius of curvature of at least 50% the width of one or both elongate portions.

Advantageously, the concave arc having such a radius of curvature provides a sufficient degree of improved conformity on contours of the wound site, and increases the absorbent pad volume proximate to the wound site without hindering the ability of the dressing to be folded abruptly (for example around a heel) or unduly increasing the bulk of the dressing.

The or each wing may have an area equal to at least 0.8% the total area of the absorbent pad. The or each wing may have an area equal to at least 0.9% the total area of the absorbent pad. The or each wing may have an area equal to at least 1% the total area of the absorbent pad. The or each wing may have an area equal to at least 1.1% the total area of the absorbent pad. The or each wing may have an area equal to at least 1.2% the total area of the absorbent pad. The or each wing may have an area equal to at least 1.3% the total area of the absorbent pad. The or each wing may have an area equal to at least 1.5% the total area of the absorbent pad. The or each wing may have an area equal to at least 2% the total area of the absorbent pad.

The or each wing may have an area of at least 1.5 cm², at least 1.6 cm², at least 1.7 cm², at least 1.8 cm², at least 1.9 cm², or at least 2.0 cm²,

Advantageously, by providing the or each wing with an area of outlined above the volume of absorbent pad proximate to the wound site is improved without unduly altering the shape of the dressing.

One, or more, or each short edge of the second elongate portion of the absorbent pad may comprise a notch. The or each notch may have an angular profile. The or each notch may have a curved profile.

The provision of notches on one or both short edges of the second elongate portion improves the ability of the second elongate portion to conform to contours in the wound site, it provides a preferential point to initiate a fold line, and improves the ability of the adjacent dressing to form a cup or dome shape. This effect is particularly pronounced where the notch has a curved profile. The curved profile has the additional benefit of improving manufacturability.

The first elongate portion may have a substantially quadrilateral perimeter. The first elongate portion may have a substantially rectangular perimeter. The first elongate portion may comprise two parallel edges. The first elongate portion may comprise two edges which taper towards one another. The first elongate portion may comprise at least two straight edges.

The second elongate portion may have a substantially quadrilateral perimeter. The second elongate portion may have a substantially rectangular perimeter. The second elongate portion may comprise two parallel edges. The second elongate portion may comprise two edges which taper towards one another. The first elongate portion may comprise at least two straight edges.

The first elongate portion, the second elongate portion and the at least one wing may define a perimeter of the absorbent pad. The first elongate portion, may define a portion of a perimeter of the absorbent pad. The second elongate portion may define a portion of a perimeter of the absorbent pad. The at least one, or each wing may define a portion of a perimeter of the absorbent pad.

The wound dressing may comprise an aperture. The aperture may be configured to connect the wound dressing to a source of non-atmospheric pressure. The aperture may be provided on the first elongate portion. The aperture may be provided on the first elongate portion distal to the second elongate portion. The aperture may be provided through a/the covering layer.

The wound dressing may comprise a wound contact layer. The wound dressing may comprise a covering layer. The wound dressing may comprise an adhesive skin contact layer. The wound dressing may comprise an absorbent layer. The wound dressing may comprise a transmission layer. The wound dressing may comprise a fluid distribution layer. The wound dressing may comprise a wicking layer. The wound dressing may comprise an absorbency layer. The wound dressing may comprise a superabsorbent layer. The wound dressing may comprise a foam layer. The wound dressing may comprise a pressure distribution layer. The wound dressing may comprise a gauze layer. The wound dressing may comprise an intralayer adhesive layer.

The absorbent pad may comprise a wound contact layer. The absorbent pad may comprise an absorbent layer. The absorbent pad may comprise a transmission layer. The absorbent pad may comprise a fluid distribution layer. The absorbent pad may comprise a wicking layer. The absorbent pad may comprise an absorbency layer. The absorbent pad may comprise a superabsorbent layer. The absorbent pad may comprise a foam layer. The absorbent pad may comprise a pressure distribution layer. The absorbent pad may comprise a gauze layer. The absorbent pad may comprise an intralayer adhesive layer.

The absorbent pad may have a "T-Shape" outline. The absorbent pad may have an "L-Shape" outline.

A/the wound contact layer may have a T-shaped outline. A/the covering layer may have a T-shaped outline. A/the adhesive skin contact layer may have a T-shaped outline. An/the absorbent layer may have a T-shaped outline. A/the transmission layer may have a T-shaped outline. A/the fluid distribution layer may have T-shaped outline. A/the wicking layer may have a T-shaped outline. A/the absorbency layer may have a T-shaped outline. A/the superabsorbent layer may have a T-shaped outline. A/the foam layer may have a T-shaped outline. A/the pressure distribution layer may have a T-shaped outline. A/the gauze layer may have a T-shaped outline. A/the intralayer adhesive layer may have a T-shaped outline. The adhesive skin contact layer may be arranged on the covering layer and have the same outline. The wound dressing (i.e. the outer periphery of the dressing as a whole) may have a T-shaped outline.

A/the wound contact layer may have a L-shaped outline. A/the covering layer may have a L-shaped outline. A/the adhesive skin contact layer may have a L-shaped outline. An/the absorbent layer may have a L-shaped outline. A/the transmission layer may have a L-shaped outline. A/the fluid distribution layer may have L-shaped outline. A/the wicking layer may have a L-shaped outline. A/the absorbency layer may have a L-shaped outline. A/the superabsorbent layer may have a L-shaped outline. A/the foam layer may have a L-shaped outline. A/the pressure distribution layer may have a L-shaped outline. A/the gauze layer may have a L-shaped outline. A/the intralayer adhesive layer may have a L-shaped outline. The adhesive skin contact layer may be arranged on the covering layer and have the same outline. The wound dressing (i.e. the outer periphery of the dressing as a whole) may have a L-shaped outline.

The first elongate portion of the absorbent pad may be tapered. The first elongate portion of the absorbent pad may reduce in with away from an interface with the second elongate portion of the absorbent pad. The taper may terminate in a rounded end.

The wound dressing may comprise at least two layers. The wound dressing may comprise a wound contact layer and a covering layer. The covering layer may extend beyond a perimeter of the wound contact layer. The covering layer may extend an equal distance beyond the perimeter of the wound contact layer about the entire perimeter. An adhesive may be provided on the border of the covering layer. An adhesive may be provided on the portion of the covering layer which extends beyond the perimeter of the wound contact layer. A region of the covering layer extending between a perimeter of the wound contact layer and a perimeter of the covering layer may be provided with an adhesive.

The wound dressing may comprise at least two layers selected from the following: a wound contacting layer, an adhesive skin contact layer, a covering layer, a fluid distribution layer, a wicking layer, an absorbency layer, a superabsorbent layer, a transmission layer, a foam layer, a pressure distribution layer, a gauze layer and/or an intralayer adhesive layer.

The wound dressing may comprise at least three layers selected from the following: a wound contacting layer, an adhesive skin contact layer, a covering layer, a fluid distribution layer, a wicking layer, an absorbency layer, a superabsorbent layer, a transmission layer, a foam layer, a pressure distribution layer, a gauze layer and/or an intralayer adhesive layer. Preferably, the wound dressing may comprise a wound contacting layer, a covering layer and one further layer listed above. More preferably the further layer is a superabsorbent layer. The further layer may be arranged between the wound contacting layer and the covering layer. The further layer may have the same outline as the wound contact layer. The further layer may have a T-shaped outline. The further layer may have an L-shaped outline. The wound contact layer and the further layer may be the same size. The wound contact layer and the further layer may be different sizes. The further layer may the larger than the wound contact layer. The further layer may be smaller than the wound contact layer. The covering layer may be the same shape as the wound contact layer and/or further layer. The covering layer may be larger than the wound contact layer and/or further layer.

The wound dressing may comprise at least four layers selected from the following: a wound contacting layer, an adhesive skin contact layer, a covering layer, a fluid distribution layer, a wicking layer, an absorbency layer, a superabsorbent layer, a transmission layer, a foam layer, a pressure distribution layer, a gauze layer and/or an intralayer adhesive layer. Preferably, the wound dressing may comprise a wound contacting layer, a covering layer and two further layers listed above. More preferably, the two further layers are a superabsorbent layer and a transmission layer. The further layers may be arranged between the wound contacting layer and the covering layer. Where the further layers are a superabsorbent layer and a transmission layer, the layers may be ordered: the wound contact layer, the transmission layer, the superabsorbent layer, and the covering layer. One or both further layers may have the same outline as the wound contact layer. The two further layers may have the same outline. The two further layers may have a different outline. One or both further layers may have a T-shaped outline. One or both further layers may have an L-shaped outline. The wound contact layer and one or both further layers may be the same size. The wound contact layer and one or both further layers may be different sizes. One or both further layers may the larger than the wound contact layer. One or both further layers may be smaller than the wound contact layer. The covering layer may be the same shape as the wound contact layer and/or one or both further layers. The covering layer may be larger than the wound contact layer and/or one or both further layers. Where the further layers are a superabsorbent layer and a transmission layer, the wound contact layer, superabsorbent layer and transmission layer may have the same T-shape and the covering layer may be a rectangular shape.

The wound dressing may comprise at least five layers selected from the following: a wound contacting layer, an adhesive skin contact layer, a covering layer, a fluid distribution layer, a wicking layer, an absorbency layer, a superabsorbent layer, a transmission layer, a foam layer, a pressure distribution layer, a gauze layer or an intralayer adhesive layer. Preferably, the wound dressing may comprise a wound contacting layer, a covering layer and three further layers listed above. More preferably the three further layers may be a superabsorbent layer, a transmission layer and an adhesive skin contact layer. The further layers may be arranged between the wound contacting layer and the covering layer. Where the further layers are a superabsorbent layer and a transmission layer and adhesive skin contact layer, the layers may be ordered: the adhesive skin contact layer, the wound contact layer, the transmission layer, the superabsorbent layer, and the covering layer. One or more or each further layer may have the same outline as the wound contact layer. Two of the three further layers may have the same outline. The three further layers may have the same outline. The three further layers may have a different outline. One or more or each further layer may have a T-shaped outline. One or more or each further layer may have an L-shaped outline. The wound contact layer and one or more or each further layer may be the same size. The wound contact layer and one or more or each further layer may be different sizes. One or more or each further layer may the larger than the wound contact layer. One or more or each further layer may be smaller than the wound contact layer. The covering layer may be the same shape as the wound contact layer and/or one or more or each further layer. The covering layer may be larger than the wound contact layer and/or one or more or each further layer.

At least two layers of the absorbent pad may be laminated together. At least two of the wound contacting layer, transmission layer, and superabsorbent layer may be laminated together. The lamination of two or more layers of the absorbent pad is advantageous because, as an example, some known wound dressings in the prior art are typically provided with an adhesive layer which creates an exterior housing or envelope with a covering layer, within which the remaining layers (for example a wound contact layer and absorbent layer) are housed. The adhesive layer is typically connected to the backing layer and the exterior surface of the absorbent structure (usually the wound contact layer) to hold the absorbent structure in place. The adhesive layer also adheres the wound dressing to the peri-wound skin. As such, the adhesive layer is commonly wetted with wound exudate during use which may reduce the adhesion between the adhesive layer and the absorbent structure. In this case, the absorbent structure may partially, or wholly, dissociate, disadvantageously affecting the application of negative pressure to the wound site and the ability of the wound dressing to retain wound exudate.

Where the wound dressing includes lamination of at least two of the wound contact layer, transmission layer and superabsorbent layer of the absorbent pad, the reliance upon an external adhesive layer to hold the absorbent structure together is decreased or removed. As such, the present invention does not succumb to the above disadvantages of the prior art.

Moreover, the present invention does not rely on additional dressing and binding components to hold the absorbent pad together, some of which require specialist manufacturing steps and techniques to incorporate into a wound dressing. The present invention uses simple lamination techniques to enhance the strength and integrity of the absorbent structure. As such, the wound dressing of the present invention is cheaper and simpler to manufacture than some wound dressings of the prior art.

Further advantageously, as the present invention does not require any relatively rigid dressing and binding components, the absorbent structure is less rigid than absorbent structures of the prior art. Thus, the provision of lamination between two or more layers of the absorbent pad provides a negative pressure wound dressing which is flexible, and in some cases more flexible than negative pressure wound dressings of the prior art and, therefore, provides greater conformation to the contours of a patient's body at a wound site in use and thus greater comfort for the patient.

Each of the wound contacting layer, the transmission layer and the superabsorbent layer may be laminated together.

The aperture may extend partially into the absorbent pad. The aperture may extend into at least one layer. The aperture may extent through at least one layer. The aperture may extend through at least one layer of the absorbent pad. The aperture may not extend into at least one layer of the absorbent pad. The aperture may not extend through at least one layer of the absorbent pad. The aperture may be provided through a/the superabsorbent layer. The aperture may be provided in a/the absorbent layer. The aperture may be provided in a/the transmission layer. The aperture may be provided in a/the fluid distribution layer. The aperture may be provided in a/the wicking layer. The aperture may be provided in a/the absorbency layer. The aperture may be provided in a/the foam layer. The aperture may be provided in a/the pressure distribution layer. The aperture may be provided in a/the gauze layer. The aperture may be provided in a/the intralayer adhesive layer.

A region of the covering layer within that defined by an/the adhesive border may be closed, sealed and/or uninterrupted. A region of the covering layer within that defined by the adhesive skin contact layer may be closed, sealed and/or uninterrupted. This restricts and or prevents microbes, bacteria and the like from entering the wound site.

The wound dressing may comprise a release layer, the release layer being removable to reveal the adhesive border or adhesive skin contact layer.

The adhesive border may comprise an adhesive skin contact layer. The adhesive skin contact layer may have a perimetral shape. The outer periphery of the adhesive skin contact layer may have the same shape of the covering layer. The adhesive skin contact layer may be provided with a window. The window may be smaller in size than the absorbent pad. The window may be the same shape as the absorbent pad. The adhesive skin contact layer may comprise a wound facing surface. The adhesive skin contact layer may comprise a wound facing surface and an opposing surface. The adhesive skin contact layer may comprise an inner region. The adhesive skin contact layer may comprise an outer region. The opposing surface in the inner region may be adhered to the absorbent pad. The opposing surface in the outer region may be adhered to the covering layer.

An outer periphery of the covering layer may define the shape of the wound dressing. The outer periphery of the covering layer may have a "T-shaped" periphery. The covering layer may comprise a first elongate portion. The covering layer may comprise a second elongate portion. A short edge of the first elongate portion (of the covering layer) may abut a long edge of the second elongate portion (of the covering layer).

Advantageously, by providing the covering layer with a T-shaped periphery the dressing can be more readily formed to highly contoured regions with reduced instances of bulging of the covering layer which could potentially increase the risk of leakage when a pressure differential is applied to the dressing.

The width of the first elongate portion (as measured along the shorter dimension of the first portion) may be between 30 and 90% the length of the second elongate portion (as measured along the longer dimension of the second portion), preferably between 40 and 80%, more preferably between 50 and 70%, such as between 60 and 70%, for example between 64 and 68%, i.e. 66%.

The length of the first elongate portion (as measured along the longer dimension of the first portion) may be between 20% and 200% the width of the second elongate portion (as measured along the shorter dimension of the second portion), preferably between 30 and 150%, more preferably between 40 and 100%, such as between 40 and 70%, for example between 45 and 55%, i.e. 50%.

An outline of the one or more or each layer(s) may have the same shape. The outline of the one or more or each layer(s) may have the same shape as the outline of the wound dressing. An outline of the one, or more, or each layer(s) may be symmetrical along a central axis.

The outline of the layer(s) and outline of the dressing having the same shape means they are geometrically similar, the relative arrangement and proportions of the two outlines are the same but they may have different sizes, for example the wound dressing outline may be uniformly scaled up with respect to the absorbent pad outline.

The wound dressing may have a length of between 120 and 320 mm, preferably between 170 and 270 mm, more preferably between 200 and 240 mm, for example between 210 and 230 mm, such as 220 mm. The wound dressing may have a width of between 120 and 460 mm, preferably between 170 and 270 mm, more preferably between 200 and 240 mm, for example between 210 and 230 mm, such as 220 mm.

The absorbent pad may have a length of between 80 and 240 mm, preferably between 120 and 200 mm, more preferably between 140 and 280 mm, for example between 150 and 170 mm, such as 160 mm.

The absorbent pad may have a maximum width of between 80 and 360 mm, preferably between 120 and 280 mm, more preferably between 140 and 180 mm, for example between 150 and 170 mm, such as 160 mm.

The first elongate portion may have a length of between 60 and 140 mm, preferably between 80 and 120 mm, more preferably between 90 and 110 mm, for example between 95 and 105 mm, such as 100 mm.

The first elongate portion may have a (where tapered, maximum) width of between 40 and 80 mm, preferably between 45 and 75mm, more preferably between 50 and 70 mm, for example between 55 and 65 mm, such as 60 mm.

The seconds elongate portion may have a length of between 60 and 360 mm, preferably between 80 and 180 mm, more preferably between 90 and 140 mm, for example between 95 and 105 mm, such as 100 mm.

The second elongate portion may have a width of between 40 and 80 mm, preferably between 45 and 75mm, more preferably between 50 and 70 mm, for example between 55 and 65 mm, such as 60 mm.

The or each notch may have a width of between 10 and 60 mm, preferably between 20 and 55 mm, more preferably between 30 and 50 mm, for example between 35 and 45 mm, such as 40 mm.

The or each notch may have a depth of between 2 and 20 mm, preferably between 5 and 15 mm, more preferably between 8 and 12 mm, such as 10 mm.

The covering layer may have a length of between 120 and 320 mm, preferably between 170 and 270 mm, more preferably between 200 and 240 mm, for example between 210 and 230 mm, such as 220 mm. The covering may have a width of between 120 and 320 mm, preferably between 170 and 270 mm, more preferably between 200 and 240 mm, for example between 210 and 230 mm, such as 220 mm.

The adhesive skin contact layer may extend at least 10 mm, at least 15 mm, at least 20 mm, at least 25 mm, at least 30 mm, at least 40 mm or at least 50 mm beyond the absorbent pad.

The covering layer may extend at least 10 mm, at least 15 mm, at least 20 mm, at least 25 mm, at least 30 mm, at least 40 mm or at least 50 mm beyond the absorbent pad.

The adhesive skin contact layer may overlap the absorbent pad by at least 2 mm, at least 4 mm, at least 6 mm, at least 8 mm, at least 10 mm, at least 12 mm, at least 15 mm, or at least 20 mm.

The absorbent pad may be arranged between a first portion of the adhesive skin contact layer and the covering layer. A second portion of the adhesive skin contact layer may be arranged on the covering layer. A second portion of the adhesive skin contact layer may be arranged on a wound facing side of the covering layer. the adhesive skin contact layer may be provide with a window. The window may be provided in the first portion of the adhesive skin contact layer.

The wound dressing may have a thickness between 1 mm to 20 mm, or 2 mm to 10 mm, or 3 mm to 7 mm, for example.

The covering layer may have a thickness of between 10 and 50 microns, preferably between 20 and 40 microns, more preferably between 25 and 35 microns, for example 30 microns.

The wound dressing may be configured for use with a source of non-atmospheric pressure. The wound dressing may be configured for use in a pressure gradient wound therapy system. The wound dressing may be configured for use in negative pressure wound therapy. The wound dressing may be configured for use in positive pressure wound therapy.

When used herein and throughout the specification the term "pressure gradient wound therapy system" is intended to cover a wound therapy system wherein a pressure differential (either positive or negative) is applied between a sealed region of the wound dressing and the surrounding environment.

As used herein, negative pressure wound therapy is a therapeutic technique using a suction dressing to remove excess exudation and promote healing in acute or chronic wounds. A vacuum of -50 to -200 mm Hg, or -75 to -150 mm Hg may be applied with typical negative pressure of -80 to -130 mm Hg, -100 to -130 mm Hg, or often about -125 mm Hg being applied to a wound.

For positive pressure wound therapy, a net positive pressure is applied to the wound, which may include providing simultaneous aspiration and irrigation of the wound. Positive pressure wound therapy may be carried out at a positive pressure of up to 50% atm., typically at a low positive pressure of up to 20% atm., more usually up to 10% atm. at the wound. Positive pressure wound therapy is known and referred to in US20180140755.

The (outer) covering layer of the dressing is provided as a bacterial and viral barrier layer which preferably resists the ingress of liquid and air but allows moisture vapour transmission. In this way the covering layer enhances the overall fluid handling capacity of the dressing by allowing for the escape of moisture vapour through the cover while enabling the application of pressure (either positive or negative) to the wound. The outer covering layer is for instance a layer having a MVTR of at least 3,000 g m⁻² per 24 hours or, more preferably in the range of from 10,000gm⁻² to 50,000g m⁻² per 24 hours measured by the method described in BS EN 13726-2 2002 "Test methods for primary wound dressings Part 2 Moisture vapour transmission rate of permeable film dressings". The covering layer may be any suitable material and may, in some embodiments, comprise a polyurethane material, as known in the art. The covering layer may have a thickness of between 25 microns and 35 microns, preferably 30 microns.

The wound dressing preferably is a one-piece dressing. That is to say, the covering layer and the body of the wound dressing are provided as an integral item, which the user or HCP is not required to assemble in-situ or before application. The wound dressing preferably including an adhesive layer and preferably including a removable release layer. The wound dressing may be provided in packaging. The packaging may form a sterile cavity in which the wound dressing is provided. The wound dressing may be integrally formed in the packaging.

Providing the wound dressing as a one-piece dressing is advantageous over the more typical NPWT dressing systems which utilise separate packing (for example foam) to contact the wound and drape to cover the wound site and provide a seal for the application of negative pressure. The one-piece system is easier for the user or HCP to apply as the layers are correctly aligned and held together.

The wound dressing may be provided in a kit. The kit may comprise at least one wound dressing and at least one sealing strip. The sealing strips may be shaped to conform to at least a portion of the perimeter of the wound dressing.

In use, the wound dressing may be connected to a source of negative pressure, for example a pump.

The covering layer may have a quadrilateral shaped periphery. In the context of the application quadrilateral is taken to encompass any four-sided shape, in particular to regular four-sided shapes such as rectangles, squares, stadiums, diamonds, more especially to rectangles and squares, including those with rounded corners such as rounded rectangles and rounded squares.

Accordingly, in a second aspect of the current disclosure there is provided a pressure gradient wound dressing, the wound dressing comprising an absorbent pad and a covering layer; the absorbent pad having a shape comprising two elongate portions arranged at an angle to one another; and the covering layer overlapping the absorbent pad and having a quadrilateral shaped periphery.

The provision of a covering layer with a quadrilateral shaped periphery for a wound dressing with an absorbent pad comprising two elongate portions arranged at an angle to one another is advantageous because it provides a large area of the covering layer which the HCP can modify without risking the integrity of the seal once the dressing is applied to the wound site. Furthermore, and in particular where the covering layer is comprised of a thin film, for example polyurethane with a thickness between 25 and 35 microns, such as 30 microns, the covering layer can readily be cut, and the two sections overlapped to improve both conformity at a contour and also the seal at that point.

The first and second elongate portions may be arranged at an angle of between 0 and 180° to one another, preferably between 30 and 150° to one another, more preferably between 45 and 135° to one another, for example between 60 and 120° to one another, such as between 75 and 105° to one another, e.g. about 90° to one another, i.e. the first and second elongate portions may be arranged perpendicular to one another.

Providing the two elongate portions at an angle to one another allows the wound dressing to be applied to cover a non-linear wound, and also wounds which extend around contoured portions of the body. It also allows an aperture through which a source of non-atmospheric pressure can be applied which is arranged away from the wound site, thereby reducing the risk of irritation of the wound site by the airway supplying the non-atmospheric pressure.

The wound dressing of the second aspect may include any of the optional features set out above in respect of the first aspect.

In some embodiments, the wound dressing of the second aspect may comprise the features of the first aspect of the invention (i.e. the wound dressing of the second aspect of the invention may comprise an absorbent pad having a shape comprising at least one wing, wherein a short edge of a first elongate portion abuts a long edge of a second elongate portion, and the at least one wing extends between a long edge of the first elongate portion and the long edge of the second elongate portion).

However, of course, the wound dressing of the second aspect of the invention may include optional features set out above in relation to the first aspect without including those features defined in respect of the first aspect of the invention. (For example, embodiments of the second aspect of the invention may include components of the absorbent pad as outlined in respect of the first aspect of the invention, but without requiring the shape of the first aspect of the invention, including the wing.)

According to a third aspect of the invention there is provided a method of applying a wound dressing according to the first or second aspects to a wound site.

The wound dressing may comprise any of the optional features of the first or second aspects of the invention.

The method may further comprise removing the wound dressing from a sterile packaging. The method may further comprise removing a release layer from the wound dressing. The method may further comprise placing a central region of the wound dressing over the wound site. The method may further comprise positioning the first elongate portion away from the wound site.

The method may further comprise connecting the wound dressing to a source of negative pressure.

According to a fourth aspect of the current disclosure there is provided a kit comprising at least one wound dressing according to the first or second aspects (including any optional features thereof) and at least one of; a port, an airway, a source of non-atmospheric pressure.

The airway may be integrally formed with the wound dressing.

According to a fifth aspect of the current disclosure there is provided a negative pressure wound therapy (NPWT) system comprising at least one wound dressing according to the first or second aspects (including any optional features thereof) connected to a pump.

It will be appreciated that, where possible, any feature or combination of features disclosed or claimed herein may be combined with any other feature or combination of features, whether or not this is explicitly described.

### Brief Description of the Drawings

In order that the invention may be more clearly understood one or more embodiments thereof will now be described, by way of example only, with reference to the accompanying drawings, of which:
Figure 1 shows a plan view of a wound dressing according to the present disclosure;
Figure 2 shows a plan view of the wound dressing of Figure 1 from the wound facing side;
Figure 3 shows a cross-sectional view of the wound dressing of Figure 1;
Figure 4 shows a perspective view of the wound dressing of Figure 1 *in situ* on a heel;
Figure 5 shows a plan view of a further wound dressing according to the present disclosure;
Figure 6 shows a cross-sectional view of the wound dressing of Figure 5;
Figure 7 shows a plan view of a further wound dressing according to the present disclosure;
Figure 8 shows a cross-sectional view of the wound dressing of Figure 7;
Figure 9 shows a plan view of a further wound dressing according to the present disclosure;
Figure 10 shows a plan view of the wound dressing of Figure 9 from the wound facing side;
Figure 11 shows a cross-sectional view of the wound dressing of Figure 9;
Figure 12 shows a perspective view of the wound dressing of Figure 9 *in situ* on a heel;
Figure 13 shows a test apparatus with the wound dressing of Figure 9 *in situ;*
Figure 14 shows a plan view of a further wound dressing according to the present disclosure;
Figure 15 shows an exploded perspective view of the dressing of Figure 14; and
Figure 16 shows a cross-sectional view of the wound dressing of Figure 14.

### Detailed Description of the Invention

In the following detailed description, numerous specific details are set forth in order to provide a thorough understanding of various embodiments and the inventive concept.

It will also be appreciated that well known methods, procedures, components, and systems may have not been described in detail.

Generally, a wound dressing may be taken to be any dressing which is applied to a wound to promote healing and protect against further deterioration of the wound. Wound dressings will typically cover and be held in direct contact with a wound. A wound dressing will generally comprise a wound contacting surface and a rear surface which faces outwards from the wound and defines an exterior of the dressing.

The wound contacting surface may be provided by a separate wound contact layer which has specific properties to promote healing. Wound contact layers are typically non-adherent layers which are placed in direct contact with the wound and may be designed to promote the removal, e.g. by wicking or absorption, of exudate. The rear surface of the dressing may comprise a separate covering layer to provide the exterior of the dressing and aforementioned rear surface. The covering layer may be configured to provide a sealed envelope against the wound to prevent unwanted contamination of the wound and allow a pressure gradient to be applied to the wound, whilst being optionally breathable to allow moisture egress to aid dressing and wound healing performance.

Wound dressings may be attached to a wound site by any known method of attachment, for example by binding with a bandage or use of adhesive tape, or with some other form of mechanical restraint which holds the dressing in place. In addition, or alternatively, the dressing may comprise an adhesive skin contact layer which is provided on the covering layer or portions of a wound contact layer so as to adhere the dressing to a wound site, as required.

In addition to the wound contact layer, it will be appreciated that many other types of layers may be included in a dressing. A dressing may comprise, for example, a covering layer for covering a wound and may further comprise one or more from the group comprising: a wound contact layer, a fluid distribution layer, a wicking layer, an absorbency layer, a superabsorbent layer, a transmission layer, a foam layer, a gauze layer, an adhesive skin contact layer or intralayer adhesive layer, amongst others. Where a wound dressing comprises a covering layer and one or more other layers, the additional layers will typically be provided in a stacked configuration in which the covering layer is provided as an outer layer which defines an external surface of the dressing which is typically adhered to the patient to cover the wound and provide an envelope in which the other layers can be housed. Each of the layers may be described in relation to the covering layer and the wound site. Thus, each layer which is located between wound site and covering layer may comprise a first surface which faces inwardly and towards the wound site, and a second surface which faces outwardly from the wound site and towards the covering layer.

The wound dressing shape described herein may be used in any type of wound dressing. The specific embodiments described below comprise a covering layer, a wound contact layer and an adhesive skin contact layer, but this is not a limitation and one or more of these layers may not be present in some examples. For example, a wound dressing according to the present disclosure may comprise a covering layer only. Such a covering layer may be used to cover a separate wound contacting dressing for example. In other examples, the wound dressing may not comprise an adhesive layer but be attached to a patient using a secondary means, such as the aforementioned bindings or the like.

The wound dressings of the present disclosure are shaped to lend themselves to multiple different types of wound sites whilst have a broadly similar shape. In doing so, the wound dressings have increased utility and require less specialist knowledge to be applied. In providing a multi-site dressing shape, it is the intention to provide a dressing which balances ease of use with increased performance in terms of wear-time and patient comfort in numerous different circumstances.

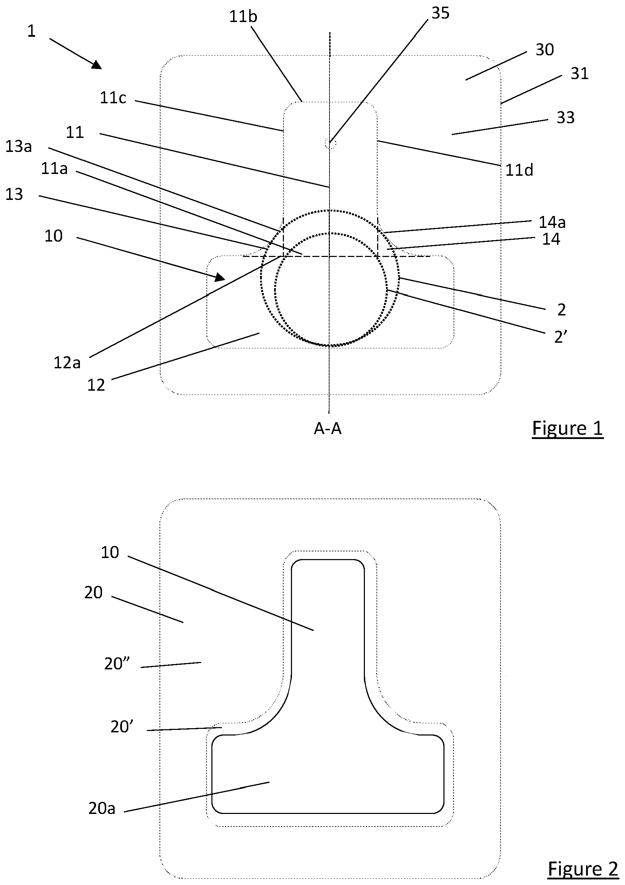

Figures 1 to 4 show a wound dressing according to a first embodiment of the present disclosure. The dressings are considered to be favourable for attaching or adhering to multiple different sites on the human body in particular to the heel or breast.

Figure 1 shows a wound dressing 1 in plan. The dressing 1 comprises an absorbent pad 10. To aid the description of the dressing 1 shape, there is shown a centre line A-A which extends centrally along the major axis of the dressing. The length of the dressing 1 is measured along the major axis and the width of the dressing 10, is measured perpendicular to the major axis.

The absorbent pad 10 has an overall "T-shape", that is it comprises a first elongate portion 11, and a second elongate portion 12. A short edge 11a of the first elongate portion 11 abuts the second elongate portion 12 perpendicularly at a midpoint of a first long edge 12a.

Extending between a first long edge 11c of the first elongate portion 11 and the long edge 12a of the second elongate portion 12 there is provided a first wing 13. The first wing 13 has a triangular shape (i.e. a generally triangular shape), with two short edges abutting the first long edge 11c of the first elongate portion 11 and the long edge 12a of the second elongate portion 12. The third edge 13a has a concavely curving profile. To aid in the understanding of the various shaped parts of the absorbent pad, dotted lines have been provided to delimit each, it will be understood these lines do not indicate any structure in the dressing.

Likewise, extending between a second long edge 11d of the first elongate portion 11 and the long edge 12a of the second elongate portion 12 there is provided a second wing 14. The second wing 14 has a triangular shape, with two short edges abutting the second long edge 11d of the first elongate portion 11 and the long edge 12a of the second elongate portion 12. The third edge 14a has a concavely curving profile.

Arranged above and extending beyond a periphery of the absorbent pad 10 is a covering layer 30, the covering layer is provided with a peripheral edge 31, the peripheral edge 31 has a square shape with rounded corners. When the wound dressing 1 is *in-situ* the covering layer 30 and wound may define a wound dressing cavity in which the remaining layers of the dressing 1 are provided. Thus, the covering layer 30 defines the shape of the dressing. The region of the covering layer 30 between the peripheral edge 31 and the periphery of the absorbent pad 10 may be referred to as a border region 33.

The covering layer 30 of the dressing is provided as a bacterial and viral barrier layer which preferably resists the ingress of liquid and air but allows moisture vapour transmission. In this way the covering layer 30 enhances the overall fluid handling capacity of the dressing by allowing for the escape of moisture vapour through the cover while enabling the application of pressure (either positive or negative) to the wound. The outer cover layer is for instance a layer having a MVTR of at least 3,000 g m⁻² per 24 hours or, more preferably, in the range of from 10,000gm⁻² to 50,000g m⁻² per 24 hours measured by the method described in BS EN 13726-2 2002 "Test methods for primary wound dressings Part 2 Moisture vapour transmission rate of permeable film dressings". The covering layer may be any suitable material and may, in some preferred embodiments, comprise a polyurethane material, as known in the art. The covering layer may have a thickness of between 25 microns and 35 microns, preferably 30 microns.

To provide fluid communication between the wound dressing cavity and a source of non-atmospheric pressure, the covering layer 30 is provided with an aperture 35. The aperture 35 is arranged over a centre line (A-A) of the first elongated portion 11 of the absorbent pad 10 approximately 75% of the length of the first elongate portion 11 distal from the interface between the first 11 and second 12 elongate portions.

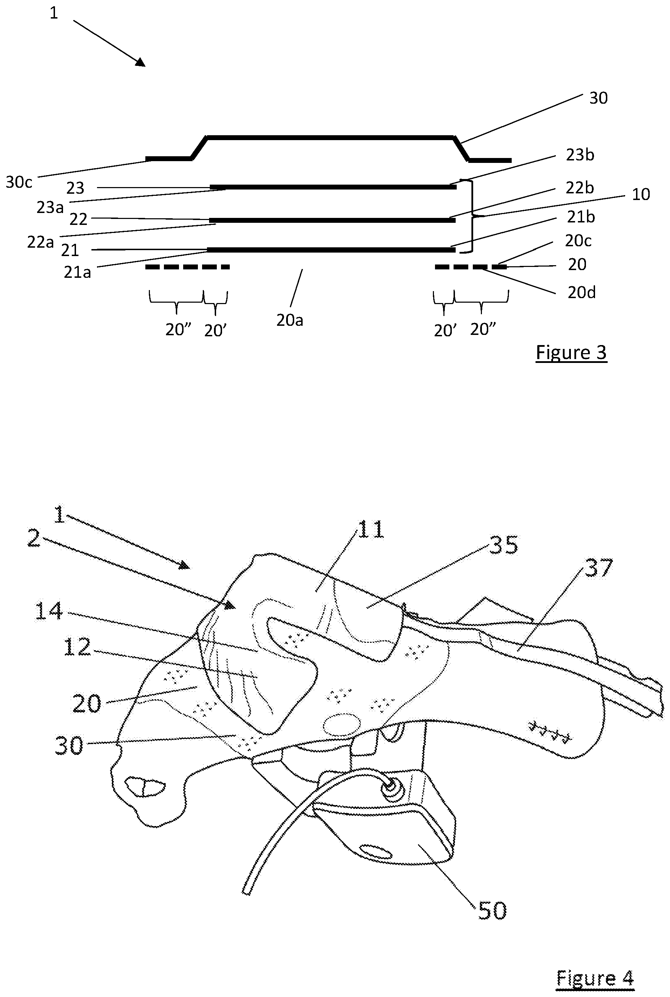

As shown in figure 3, the absorbent pad may be comprised of a plurality of layers. In order from the wound (or peri-wound area) to the covering layer 30 these may be an adhesive skin contact layer 20, a wound contact layer 21, a transmission layer 22, and a superabsorbent layer 23. In some embodiments the wound contact layer 21, the transmission layer 22, and the superabsorbent layer 23 may be laminated together, this means each of the layers form a consolidated, absorbent and retentive structure. Advantageously, this reduces or removed the need for additional components to maintain the structure, thereby reducing the bulk of the dressing and improving flexibility and further enhancing the ability of the dressing to conform to contoured wound sites.

The adhesive skin contact layer 20 may have a perimetral shape with a central window 20a, the outer periphery 20b of the adhesive skin contact layer may be contiguous with the peripheral edge 31 of the covering layer 30. The window 20a is a through hole in the adhesive skin contact layer, it is the same shape as the absorbent pad, but is sized such that it is smaller than the absorbent pad 10. As such the adhesive skin contact layer 20 retains the absorbent pad 10 in position between the adhesive skin contact layer 20 and the covering layer 30.

The adhesive skin contact layer 20 comprises an upper surface 20c and a lower surface 20d. The lower surface 20d contacts the dermal surface in use. In use, the lower surface 20d of the adhesive skin contact layer 20 is detachably adhered to a dermal surface (not shown) of a patient such that the wound site (not shown) is located within the window 20a. As such, the adhesive skin contact layer 20 is not in direct contact with the wound site but does wholly surround it. Thus, the adhesive skin contact layer 20 is in contact with intact peri-wound skin only. The upper surface 20c may be outwardly facing away from the patient's body.

A removable cover or "release layer" (not shown) may be adhered to the lower surface 20d of the adhesive skin contact layer 20. The removable cover, which may comprise folded grip sections, is removable from the lower surface 20d of the adhesive skin contact layer 20. Thus, the removable cover protects the adhesive skin contact layer 20 when the removable cover is adhered to the adhesive skin contact layer 20, but when the removable cover is removed from the lower surface 20d of the adhesive skin contact layer 20 for use of the wound dressing 1, the lower surface 20d is exposed and able to releasably secure the wound dressing 1 to the skin of a patient.

The wound contact layer 21 may comprise a first surface 21a and a second surface 21b. In use, the first surface 21a may be inwardly facing toward the patient's body, and the second surface 21b may be outwardly facing away from the patient's body. The first surface 21a of the wound contact layer 21 may contact the wound when the wound dressing 1 is adhered to the skin adjacent to the wound (i.e., when in use). The first surface 21a of the wound contact layer 21 is substantially aligned with the window 20a of the adhesive skin contact layer 20. As such, in use, the first surface 21a contacts the wound through the window 20a. The second surface 21b of the wound contact layer 21 may be adjacent, and in contact with, a first surface 22a of the transmission layer 22 or a first surface 23a of the superabsorbent layer 23. In the described embodiment, the second surface 22b of the wound contact layer 22 is adjacent, and in contact with, the first surface 23a of the transmission layer 23.

The transmission layer 22 may comprise a first surface 22a and a second surface 22b. In use, the first surface 22a may be inwardly facing toward the patient's body, and the second surface 22b may be outwardly facing away from the patient's body. The first surface 22a of the transmission layer 22 may be adjacent, and in contact with, the second surface 21b of the wound contact layer 21 or a second surface 23b of the superabsorbent layer 23. The second surface 22b of the transmission layer 22 may be adjacent, and in contact with, a first surface 23a of the superabsorbent layer 23 or the first surface 30c of the covering layer 30. In the described embodiment, the first surface 22a of the transmission layer 22 is adjacent, and in contact with, the second surface 21b of the wound contact layer, and the second surface 22b of the transmission layer 22 is adjacent, and in contact with, the first surface 23a of the superabsorbent layer 23.

The superabsorbent layer 23 may comprise a first surface 23a and a second surface 23b. In use, the first surface 23a may be inwardly facing toward the patient's body, and the second surface 23b may be outwardly facing away from the patient's body. The first surface 23a of the superabsorbent layer 23 may be adjacent, and in contact with, the second surface 21b of the wound contact layer 21 or the second surface 22b of the transmission layer 22. The second surface 23b of the superabsorbent layer 23 may be adjacent, and in contact with, the first surface 22a of the transmission layer 22 or the first surface 30c of the backing layer 30. In the described embodiment, the first surface 23a of the superabsorbent layer 23 is adjacent, and in contact with, the second surface 22b of the transmission layer 22, and the second surface 23b of the superabsorbent layer 23 is adjacent, and in contact with, the first surface 30c of the backing layer 30.

The adhesive skin contact layer 20 may radially overlap the absorbent pad 10. The adhesive skin contact layer 20 may comprise an interior portion 20' adjacent to and surrounding the window 20a, and an exterior portion 20" radially outwardly from the interior portion. The interior portion and the exterior portion of the first surface 20c of the adhesive skin contact layer 20 may contact and adhere to the skin of a patient in use. The interior portion of the second surface 20d of the adhesive skin contact layer 20 may be adjacent, and in contact with, a portion of the first surface 21a of the wound contact layer 21. The exterior portion of the second surface 20b of the adhesive skin contact layer 20 may be adjacent, and joined with, a peripheral portion of the first surface 30c of the backing layer 30. Beneficially, this helps to prevent wound exudate escaping from the confines of the wound dressing which would be unhygienic and cause discomfort for the user. Moreover, this also helps maintain a negative pressure at the wound site. Furthermore, it helps maintain the structure of the dressing prior to use, meaning the dressing is integrally formed as a "one-piece" dressing.

The exterior portion of the second surface 20d of the adhesive skin contact layer 20 may be joined to the peripheral portion of the first surface 30c of the backing layer 30 by heat lamination, adhesive, welding or stitching.

The exterior portion of the second surface 20d of the adhesive skin contact layer 20 may extend radially beyond the periphery of the absorbent structure by about 12 mm.

A central region 2 of the dressing 1 may generally refer to the area in which a wound would be located when in use, i.e. within a region of the wound contact layer 21. In some embodiments the central region 2 of the dressing may refer to a circular (or elliptical) region arranged at the interface between the first 10 and second 11 elongate portions of the absorbent pad 10 and encompassing some or all of the wings 12,13. It will be appreciated however that the layers of the wound dressing in the central region 2 does not have a different composition to the remainder of the same layer. The provision of wings 12,13 greatly increases the size of the central region 2, as can be seen in Figure 1, a circular central region 2' bounded by the corners of the first and second elongate members has an area substantially smaller than a circular central region 2 bound by the wings 12,13. Indeed, in the present embodiment, the wings 12,13 represent a modest 4% increase in surface area of the absorbent pad 10, however they provide an approximately 40% increase in the area of the central region. This allows the wound dressing 1 to be used on larger wounds and/or reduces the accuracy with which the dressing needs to be applied to the wound site.

The wound dressing 1 is shaped to conform more readily to different areas of a patient's body which are typically but not necessarily contoured and/or difficult areas to adhere a dressing to. For example, these areas may include a shoulder, armpit, elbow, wrist, sacral region, hip and in particular the breast and heel.

In this embodiment the wound dressing 1 has a length (as measured between two opposing peripheral edges of the covering layer 30) of 220 mm, and a width (as measured between the other two opposing peripheral edges of the covering layer) of 220 mm.

In this embodiment the first elongate portion has a length of 100 mm and a width of 60 mm, and the second elongate portion has a length of 160 mm and a width of 60 mm therefor, the length of the absorbent pad 10, which comprises the length of the first elongate portion and the width of the second elongate portion is 160 mm. The maximum width of the absorbent pad 10 is also 160 mm, as measured along the length of the second elongate portion. The concavely curved edge 13a,14a of each wing 13,14 has a radius of curvature of 30 mm.

The convex corners of the absorbent pad 10, that is the two corners of the first elongate portion 11 distal to the wings 12,14 and the four corners of the second elongate portion are rounded with a radius of 10 mm.

The absorbent pad 10 is arranged centrally within the wound dressing 1, due to the different shapes of the absorbent pad 10 the covering layer 30 the width of the border region 33 (as measured perpendicular from the periphery of the absorbent pad 10 to the peripheral edge 31 of the covering layer 30) is not uniform. Minima in the width of the border region 33 are found adjacent to a number of points; a second short edge of the first elongate portion 11b, and the two short edges 12c,d and second long edge 12b of the second elongate portion 12. At these points the border region has a width of 30 mm.

The interior region 20' of the adhesive skin contact layer 20 was a width of 12 mm, this provides a sufficient degree of retention of the absorbent pad 12 without hindering the effectiveness thereof.

Of course, the aforementioned teachings could be applied to dressings of other sizes. For example, the dressing could be uniformly scaled down for use with smaller wound sites, or uniformly scaled up for larger wound sites. Equally, the dressing may be non-uniformly scaled, thereby providing a more elongate dressing or a wider dressing as required to suit a particular wound site whilst still retaining the T-shaped outline of the absorbent pad 10. Likewise, the width of the border region could be varied, for example from between 10 mm and 50 mm with a wider border region providing greater adhesion to the wound site and an improved seal for the wound dressing cavity.

With reference to Figure 4, in use the wound dressing 1 is applied to the wound site such that the central region 2 is arranged over the wound site and is secured in place using the adhesive skin contact layer 20. In the present example the dressing 1 is applied to a patient with a wound on their heel. The central region 2 is applied over the wound with the distal ends of the second elongate portion 12c,d being folded onto the sides of the foot, the wings 13,14 help the wound dressing 1 conform to this fold and also provide an increased volume of absorbent pad 10 proximate to the wound, this provides improved fluid management at the wound site. The first elongate region 11 is folded around the heel and extends up the back of the ankle. The shape of the wound dressing 1 lends itself to being folded as described above. When folded the border region 33, in particular the portion outward of the wings 13,14 may bulge, due to the increased amount of covering layer 30 in the portion, a HCP can readily cut this part to assist in conforming the dressing to the wound site without risk of compromising the seal, indeed the large amount of material lends itself to being overlapped once cut, thereby improving the seal. This effect is particularly pronounced in a preferred embodiment where the covering layer 30 and adhesive skin contact layer 20 comprises flexible film adhesive constructs (for example a polyurethane film with a silicone adhesive), , which are easily cut and respond well to being overlapped.

Next an airway 37 is attached to the aperture 35 connecting the dressing 1 to a source of non-atmospheric pressure 50 (for example, when configured for use in NPWT, a pump). To further improve the seal sealing strips (not shown) may applied to the perimeter of the dressing 1 to ensure that there are no leaks. The provision of the aperture 35 over the first elongate portion 11 of the absorbent pad 10 is particularly advantageous as it is beneficial to the user that the connection to the non-atmospheric pressure (e.g. or airway 37) is not arranged above the wound site. In instances where the port is provided above the wound site an external application of pressure onto the dressing, for example where the user lies on it or rests against it, applies greater pressure at the port due to it raised nature.

In other instances, in particular where the wound is linear, for example as a result of an incision, the wound dressing 1 can be arranged such that the second elongate portion 12 extends along the wound, in such instances the wings again provide an increase in absorbent pad volume proximate to the wound site.

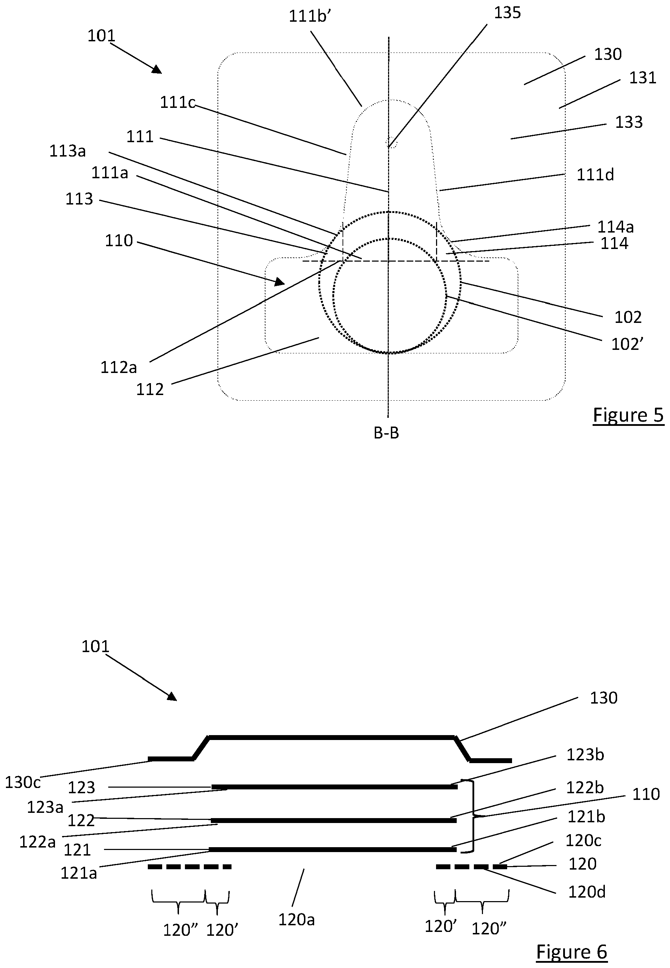

With reference to Figure 5 and 6 a wound dressing 101 according to a second embodiment of the invention is shown. The wound dressing of the second embodiment bears a number of similarities to the dressing 1 of the first embodiment, with like features having the same numerals, advanced by 100. As compared to the dressing 1 of the first embodiment, the wound dressing 101 of the second embodiment provides improved efficiency to the application of non-atmospheric pressure.

Figure 5 shows a wound dressing 101 in plan. The dressing 101 comprises an absorbent pad 110. To aid the description of the dressing 101 shape, there is shown a centre line B-B which extends centrally along the major axis of the dressing. The length of the dressing 101 is measured along the major axis and the width of the dressing 110, is measured perpendicular to the major axis.

The absorbent pad 110 has an overall "T-shape", that is it comprises a first elongate portion 111, and a second elongate portion 112. A short edge 111a of the first elongate portion 111 abuts the second elongate portion 112 perpendicularly at a midpoint of a first long edge 112a.

Extending between a first long edge 111c of the first elongate portion 111 and the long edge 112a of the second elongate portion 112 there is provided a first wing 113. The first wing 113 has a triangular shape, with two short edges abutting the first long edge 111c of the first elongate portion 111 and the long edge 112a of the second elongate portion 112. The third edge 113a has a concavely curving profile.

Likewise, extending between a second long edge 111d of the first elongate portion 111 and the long edge 112a of the second elongate portion 112 there is provided a second wing 114. The first wing 113 has a triangular shape, with two short edges abutting the second long edge 111d of the first elongate portion 111 and the long edge 112a of the second elongate portion 112. The third edge 114a has a concavely curving profile. To aid in the understanding of the various parts of the absorbent pad, dotted lines have been provided to delimit each, it will be understood these lines do not indicate any structure in the dressing.

As will be discussed in greater detail below, the dressing 101 will typically be placed such that the second elongate portion 112 of the absorbent pad, or the region at the interface between the first 111 and second 112 elongate portions is arranged over the wound. The end of the first elongate portion 111 distal to the second elongate portion 112 plays a reduced role in extrudate management, as such it is beneficial to reduce the volume of this area thereby improving the efficiency of the delivery of non-atmospheric pressure. To this end the first elongate portion 111 tapers the width decreasing away from the interface with the second elongate portion 112 and has a rounded distal end 111b'.

Arranged above and extending beyond a periphery of the absorbent pad 110 is a covering layer 130, the covering layer is provided with a peripheral edge 131, the peripheral edge 131 has a square shape with rounded corners. The covering layer 130 and wound may define a wound dressing cavity in which the remaining layers of the dressing 101 are provided. Thus, the covering layer 130 defines the shape of the dressing. The region of the covering layer 130 between the peripheral edge 131 and the periphery of the absorbent pad may be referred to as a border region 133.

The covering layer 130 of the dressing is provided as a bacterial and viral barrier layer which preferably resists the ingress of liquid and air but allows moisture vapour transmission. In this way the covering layer 130 enhances the overall fluid handling capacity of the dressing by allowing for the escape of moisture vapour through the cover while enabling the application of pressure (either positive or negative) to the wound. The outer cover layer is for instance a layer having a MVTR of at least 3,000 g m⁻² per 24 hours or, more preferably, in the range of from 10,000gm⁻² to 50,000g m⁻² per 24 hours measured by the method described in BS EN 13726-2 2002 "Test methods for primary wound dressings Part 2 Moisture vapour transmission rate of permeable film dressings". The covering layer may be any suitable material and may, in some embodiments, comprise a polyurethane material, as known in the art. The covering layer may have a thickness of between 25 microns and 35 microns, preferably 30 microns.

To provide fluid communication between the wound dressing cavity and a source of non-atmospheric pressure, the covering layer 130 is provided with an aperture 135. The aperture 135 is arranged over a centre line of the first elongated portion 111 of the absorbent pad 110 approximately 75% of the length of the first elongate portion 111 distal from the interface between the first 111 and second 112 elongate portions.

As in the first embodiment, the absorbent pad may be comprised of a plurality of layers. In order from the wound (or peri-wound area) to the covering layer 130 these may be an adhesive skin contact layer 120, a wound contact layer 121, a transmission layer 122, and a superabsorbent layer 123.

In some embodiments the wound contact layer 121, the transmission layer 122, and the superabsorbent layer 123 may be laminated together, this means each of the layers form a consolidated, absorbent and retentive structure. Advantageously, this reduces or removed the need for additional components to maintain the structure, thereby reducing the bulk of the dressing and improving flexibility and further enhancing the ability of the dressing to conform to contoured wound sites.

The adhesive skin contact layer 120 may have a perimetral shape with a central window 120a, the outer periphery 120b of the adhesive skin contact layer may be contiguous with the peripheral edge 131 of the covering layer 130. The window 120a is a through hole in the adhesive skin contact layer, it is the same shape as the absorbent pad, but is sized such that it is smaller than the absorbent pad 110. As such the adhesive skin contact layer 120 retains the absorbent pad 110 in position between the adhesive skin contact layer 120 and the covering layer 130.

The adhesive skin contact layer 120 comprises an upper surface 120c and a lower surface 120d. The lower surface 120d contacts the dermal surface in use. In use, the lower surface 120d of the adhesive skin contact layer 120 is detachably adhered to a dermal surface (not shown) of a patient such that the wound site (not shown) is located within the window 120a. As such, the adhesive skin contact layer 120 is not in direct contact with the wound site but does wholly surround it. Thus, the adhesive skin contact layer 120 is in contact with intact peri-wound skin only. The upper surface 120b may be outwardly facing away from the patient's body.

A removable cover or "release layer" (not shown) may be adhered to the lower surface 120d of the adhesive skin contact layer 120. The removable cover, which may comprise folded grip sections, is removable from the lower surface 120d of the adhesive skin contact layer 120. Thus, the removable cover protects the adhesive skin contact layer 120 when the removable cover is adhered to the adhesive skin contact layer 120, but when the removable cover is removed from the lower surface 120d of the adhesive skin contact layer 120 for use of the wound dressing 101, the lower surface 120d is exposed and able to releasably secure the wound dressing 101 to the skin of a patient.

The wound contact layer 21 may comprise a first surface 121a and a second surface 121b. **In** use, the first surface 121a may be inwardly facing toward the patient's body, and the second surface 121b may be outwardly facing away from the patient's body. The first surface 121a of the wound contact layer 121 may contact the wound when the wound dressing 101 is adhered to the skin adjacent to the wound (i.e., when in use). The first surface 121a of the wound contact layer 121 is substantially aligned with the window 120a of the adhesive skin contact layer 120. As such, in use, the first surface 121a contacts the wound through the window 120a. The second surface 121b of the wound contact layer 121 may be adjacent, and in contact with, a first surface 122a of the transmission layer 122 or a first surface 123a of the superabsorbent layer 123. In the described embodiment, the second surface 122b of the wound contact layer 122 is adjacent, and in contact with, the first surface 123a of the transmission layer 123.

The transmission layer 122 may comprise a first surface 122a and a second surface 122b. In use, the first surface 122a may be inwardly facing toward the patient's body, and the second surface 122b may be outwardly facing away from the patient's body. The first surface 122a of the transmission layer 122 may be adjacent, and in contact with, the second surface 121b of the wound contact layer 121 or a second surface 123b of the superabsorbent layer 123. The second surface 122b of the transmission layer 122 may be adjacent, and in contact with, a first surface 123a of the superabsorbent layer 123 or the first surface 130c of the covering layer 130. In the described embodiment, the first surface 122a of the transmission layer 122 is adjacent, and in contact with, the second surface 121b of the wound contact layer, and the second surface 122b of the transmission layer 122 is adjacent, and in contact with, the first surface 123a of the superabsorbent layer 123.

The superabsorbent layer 123 may comprise a first surface 123a and a second surface 123b. In use, the first surface 123a may be inwardly facing toward the patient's body, and the second surface 123b may be outwardly facing away from the patient's body. The first surface 123a of the superabsorbent layer 123 may be adjacent, and in contact with, the second surface 121b of the wound contact layer 121 or the second surface 122b of the transmission layer 122. The second surface 123b of the superabsorbent layer 123 may be adjacent, and in contact with, the first surface 122a of the transmission layer 122 or the first surface 130c of the backing layer 130. In the described embodiment, the first surface 123a of the superabsorbent layer 123 is adjacent, and in contact with, the second surface 122b of the transmission layer 122, and the second surface 123b of the superabsorbent layer 123 is adjacent, and in contact with, the first surface 130c of the backing layer 130.

The adhesive skin contact layer 120 may radially overlap the absorbent pad 110. The adhesive skin contact layer 120 may comprise an interior portion 120' adjacent to and surrounding the window 120a, and an exterior portion 120" radially outwardly from the interior portion. The interior portion and the exterior portion of the first surface 120c of the adhesive skin contact layer 120 may contact and adhere to the skin of a patient in use. The interior portion of the second surface 120d of the adhesive skin contact layer 120 may be adjacent, and in contact with, a portion of the first surface 121a of the wound contact layer 121. The exterior portion of the second surface 120b of the adhesive skin contact layer 120 may be adjacent, and joined with, a peripheral portion of the first surface 130c of the backing layer 130. Beneficially, this helps to prevent wound exudate escaping from the confines of the wound dressing which would be unhygienic and cause discomfort for the user. Moreover, this also helps maintain a negative pressure at the wound site. Furthermore, it maintains the structure of the dressing prior to use, meaning the dressing is integrally formed as a "one-piece" dressing.

The exterior portion (or border region) of the second surface 120d of the adhesive skin contact layer 120 may be joined to the peripheral portion of the first surface 130c of the backing layer 130 by heat lamination, adhesive, welding or stitching.

The exterior portion of the second surface 120d of the adhesive skin contact layer 120 may extend radially beyond the periphery of the absorbent structure by about 12 mm.

A central region 102 of the dressing 101 may generally refer to the area in which a wound would be located when in use, i.e. within a region of the wound contact layer 121. In some embodiments the central region of the dressing may refer to a circular (or elliptical) region arranged at the interface between the first 110 and second 111 elongate portions of the absorbent pad 110 and encompassing some or all of the wings 112,113. It will be appreciated however that the layers of the wound dressing in the central region 102 does not have a different composition to the remainder of the same layer.

The provision of wings 112,113 greatly increases the size of the central region 102, as can be seen in Figure 5, a circular central region 102' bounded by the corners of the first and second elongate members has an area substantially smaller than a circular central region 102 bound by the wings 112,113. Indeed, in the present embodiment, the wings 112,113 represent a modest 4% increase in surface area of the absorbent pad 110, however they provide an approximately 40% increase in the area of the central region 102. This allows the wound dressing 101 to be used on larger wounds and/or reduces the accuracy with which the dressing needs to be applied to the wound site.

The wound dressing 101 is shaped to conform more readily to different areas of a patient's body which are typically but not necessarily contoured and/or difficult areas to adhere a dressing to. For example, these areas may include a shoulder, armpit, elbow, wrist, sacral region, hip and in particular the breast and heel.

In this embodiment the wound dressing 1001 has a length (as measured between two opposing peripheral edges of the covering layer along B-B) of 220 mm, and a width (as measured between the other two opposing peripheral edges of the covering layer) of 220 mm.

In this embodiment the first elongate portion has a length of 100 mm and a width of 60 mm and the second elongate portion has a length of 160 mm and a width tapering from 60 mm proximate to the wings 113,114 to 50 mm proximate to the aperture 35. The length of the absorbent pad 110, which comprises the length of the first elongate portion and the width of the second elongate portion is 160 mm. The maximum width of the absorbent pad 110 is also 160 mm, as measured along the length of the second elongate portion. The concavely curved edge 113a,114a of each wing 113,114 has a radius of curvature of 30 mm.

The distal end 111c of the first elongate portion 111 of the absorbent pad 110 is rounded with a radius of 25 mm and thus the distal end 111c terminates as a semicircular profile, this further reduces the volume of the first elongate portion. The convex corners of the second elongate portion are rounded with a radius of 10 mm.

The absorbent pad 110 is arranged centrally within the wound dressing 101, due to the different shapes of the absorbent pad 110 the covering layer 130 the width of the border region 133 (as measured perpendicular from the periphery of the absorbent pad 110 to the peripheral edge 131 of the covering layer 130) is not uniform. Minima in the width of the border region 133 are found adjacent to a number of points; a second short edge of the first elongate portion 111b, and the two short edges 112c,d and second long edge 112b of the second elongate portion 112. At these points the border region has a width of 30 mm.

The interior region 120' of the adhesive skin contact layer 120 was a width of 12 mm, this provides a sufficient degree of retention of the absorbent pad 112 without hindering the effectiveness thereof.

Of course, the aforementioned teachings could be applied to dressings of other sizes. For example, the dressing could be uniformly scaled down for use with smaller wound sites, or uniformly scaled up for larger wound sites. Equally, the dressing may be non-uniformly scaled, thereby providing a more elongate dressing or a wider dressing as required to suit a particular wound site whilst still retaining the overall T-shaped outline of the absorbent pad 110. Likewise, the width of the border region could be varied, for example from between 10 mm and 50 mm with a wider border region providing greater adhesion to the wound site and an improved seal for the wound dressing cavity.

In use the wound dressing 101 is applied to the wound site such that the central region 102 is arranged over the wound site and is secured in place using the adhesive skin contact layer 120. The central region 102 is applied over the wound being folded and/or bent to conform to the wound site. The wings 113,114 help the wound dressing 101 achieve this conformation and also provide an increased volume of absorbent pad 110 proximate to the wound, this provides improved fluid management at the wound site. The first elongate region 111 extends away from the wound site. When the dressing 101 is folded or flexed the border region 133, in particular the portion outward of the wings 113,114 may bulge, due to the increased amount of covering layer 130 in the portion, a HCP can readily cut this part to assist in conforming the dressing to the wound site without risk of compromising the seal, indeed the large amount of material lends itself to being overlapped once cut, thereby improving the seal. This effect is particularly pronounced in a preferred embodiment where the covering layer 13 0 and adhesive skin contact layer 120 comprises flexible film adhesive constructs (for example a polyurethane film with a silicone adhesive),, which are easily cut and respond well to being overlapped.

Next the wound dressing 101 is attached to a source of non-atmospheric pressure (for example, when configured for use in NPWT, a pump) via an airway 137. To further improve the seal sealing strips may applied to the perimeter of the dressing 101 to ensure that there are no leaks. The provision of the aperture 135 over the first elongate portion 111 of the absorbent pad 110 is particularly advantageous as it is beneficial to the user that the connection to the non-atmospheric pressure (i.e. airway) is not arranged above the wound site. In instances where the port is provided above the wound site an external application of pressure onto the dressing, for example where the user lies on it or rests against it, applies greater pressure at the port due to it raised nature.

In further instances, in particular where the wound is linear, for example as a result of an incision, the wound dressing 101 can be arranged such that the second elongate portion 112 extends along the wound, in such instances the wings 113,114 again provide an increase in absorbent pad 110 volume proximate to the wound site.

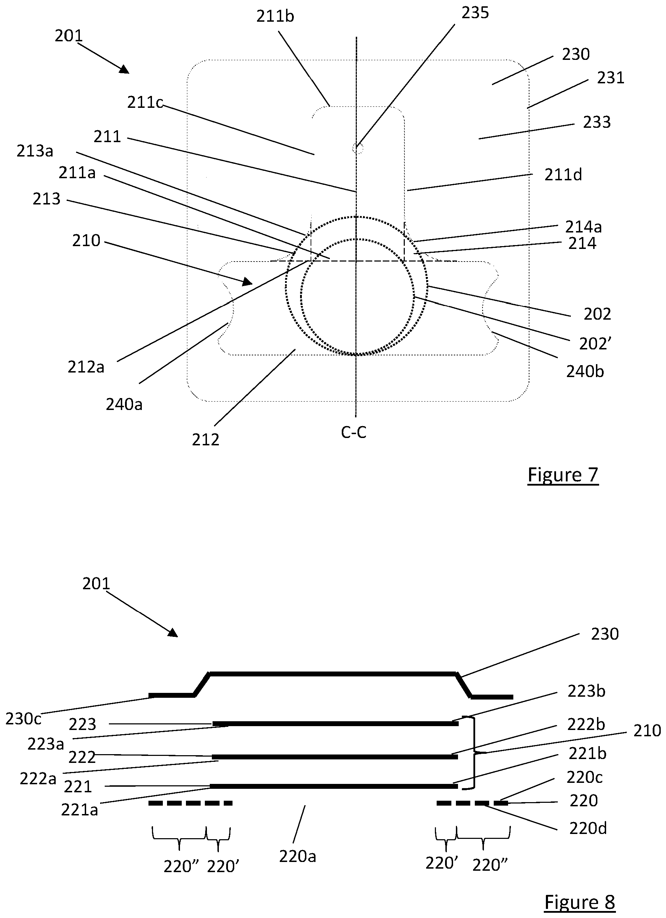

With reference to Figures 7 and 8 a wound dressing 201 according to a third embodiment of the invention is shown. The wound dressing of the third embodiment bears a number of similarities to the dressing 1 of the first and second embodiments, with like features having the same numerals, advanced by 200 and 100 respectively. As compared to the first and second embodiments the wound dressing 201 of the third embodiment provides improve conformality on contoured wound sites.

Figure 7 shows the wound dressing 201 in plan. The dressing 201 comprises an absorbent pad 210. To aid the description of the dressing 201 shape, there is shown a centre line C-C which extends centrally along the major axis of the dressing. The length of the dressing 201 is measured along the major axis and the width of the dressing 201, is measured perpendicular to the major axis.

The absorbent pad 210 has an overall "T-shape", that is it comprises a first elongate portion 211, and a second elongate portion 212. A short edge 211a of the first elongate portion 211 abuts the second elongate portion 212 perpendicularly at a midpoint of a first long edge 212a.

Extending between a first long edge 211c of the first elongate portion 211 and the long edge 212a of the second elongate portion 212 there is provided a first wing 213. The first wing 213 has a triangular shape, with two short edges abutting the first long edge 211c of the first elongate portion 211 and the long edge 212a of the second elongate portion 212. The third edge 213a has a concavely curving profile.

Likewise, extending between a second long edge 211d of the first elongate portion 211 and the long edge 212a of the second elongate portion 212 there is provided a second wing 214. The second wing 214 has a triangular shape, with two short edges abutting the second long edge 211d of the first elongate portion 211 and the long edge 212a of the second elongate portion 212. The third edge 214a has a concavely curving profile.

To improve the ability of the dressing for conform to the contours of the wound site, the short edges 212c,d are provided with a notch 240a,b. Each notch 240a,b has a curved profile with a depth one quarter the width of the notch.

Arranged above and extending beyond a periphery of the absorbent pad 210 is a covering layer 230, the covering layer is provided with a peripheral edge 231, the peripheral edge 231 has a square shape with rounded corners. The covering layer 230 and wound may define a wound dressing cavity in which the remaining layers of the dressing 201 are provided. Thus, the covering layer 230 defines the shape of the dressing. The region of the covering layer 230 between the peripheral edge 231 and the periphery of the absorbent pad may be referred to as a border region 233.

The covering layer 230 of the dressing is provided as a bacterial and viral barrier layer which preferably resists the ingress of liquid and air but allows moisture vapour transmission. In this way the covering layer 230 enhances the overall fluid handling capacity of the dressing by allowing for the escape of moisture vapour through the cover while enabling the application of pressure (either positive or negative) to the wound. The outer cover layer is for instance a layer having a MVTR of at least 3,000 g m⁻² per 24 hours or, more preferably, in the range of from 10,000gm⁻² to 50,000g m⁻² per 24 hours measured by the method described in BS EN 13726-2 2002 "Test methods for primary wound dressings Part 2 Moisture vapour transmission rate of permeable film dressings". The covering layer may be any suitable material and may, in some embodiments, comprise a polyurethane material, as known in the art. The covering layer may have a thickness of between 25 microns and 35 microns, preferably 30 microns.

To provide fluid communication between the wound dressing cavity and a source of non-atmospheric pressure, the covering layer 230 is provided with an aperture 235. The aperture 235 is arranged over a centre line of the first elongated portion 211 of the absorbent pad 210 approximately 75% of the length of the first elongate portion 211 distal from the interface between the first 211 and second 212 elongate portions.

As shown in Figure 8, the absorbent pad may be comprised of a plurality of layers. In order from the wound (or peri-wound area) to the covering layer 230 these may be an adhesive skin contact layer 220, a wound contact layer 221, a transmission layer 222, and a superabsorbent layer 223.

In some embodiments the wound contact layer 221, the transmission layer 222, and the superabsorbent layer 223 may be laminated together, this means each of the layers form a consolidated, absorbent and retentive structure. Advantageously, this reduces or removed the need for additional components to maintain the structure, thereby reducing the bulk of the dressing and improving flexibility and further enhancing the ability of the dressing to conform to contoured wound sites.

The adhesive skin contact layer 220 may have a perimetral shape with a central window 220a, the outer periphery 220b of the adhesive skin contact layer may be contiguous with the peripheral edge 231 of the covering layer 230. The window 220a is a through hole in the adhesive skin contact layer, it is the same shape as the absorbent pad, but is sized such that it is smaller than the absorbent pad 210. As such the adhesive skin contact layer 220 retains the absorbent pad 210 in position between the adhesive skin contact layer 220 and the covering layer 230.

The adhesive skin contact layer 220 comprises an upper surface 220c and a lower surface 220d. The lower surface 220d contacts the dermal surface in use. In use, the lower surface 220d of the adhesive skin contact layer 220 is detachably adhered to a dermal surface (not shown) of a patient such that the wound site (not shown) is located within the window 220a. As such, the adhesive skin contact layer 220 is not in direct contact with the wound site but does wholly surround it. Thus, the adhesive skin contact layer 220 is in contact with intact peri-wound skin only. The upper surface 220b may be outwardly facing away from the patient's body.

A removable cover or "release layer" (not shown) may be adhered to the lower surface 220d of the adhesive skin contact layer 220. The removable cover, which may comprise folded grip sections, is removable from the lower surface 20d of the adhesive skin contact layer 220. Thus, the removable cover protects the adhesive skin contact layer 220 when the removable cover is adhered to the adhesive skin contact layer 220, but when the removable cover is removed from the lower surface 220d of the adhesive skin contact layer 220 for use of the wound dressing 1, the lower surface 220d is exposed and able to releasably secure the wound dressing 1 to the skin of a patient.

The wound contact layer 221 may comprise a first surface 221a and a second surface 221b. In use, the first surface 221a may be inwardly facing toward the patient's body, and the second surface 221b may be outwardly facing away from the patient's body. The first surface 21a of the wound contact layer 221 may contact the wound when the wound dressing 201 is adhered to the skin adjacent to the wound (i.e., when in use). The first surface 221a of the wound contact layer 221 is substantially aligned with the window 220a of the adhesive skin contact layer 220. As such, in use, the first surface 221a contacts the wound through the window 220a. The second surface 221b of the wound contact layer 221 may be adjacent, and in contact with, a first surface 222a of the transmission layer 222 or a first surface 223a of the superabsorbent layer 223. In the described embodiment, the second surface 222b of the wound contact layer 222 is adjacent, and in contact with, the first surface 223a of the transmission layer 223.

The transmission layer 222 may comprise a first surface 222a and a second surface 222b. In use, the first surface 222a may be inwardly facing toward the patient's body, and the second surface 222b may be outwardly facing away from the patient's body. The first surface 222a of the transmission layer 222 may be adjacent, and in contact with, the second surface 221b of the wound contact layer 221 or a second surface 223b of the superabsorbent layer 223. The second surface 222b of the transmission layer 222 may be adjacent, and in contact with, a first surface 223a of the superabsorbent layer 223 or the first surface 230c of the covering layer 230. In the described embodiment, the first surface 222a of the transmission layer 222 is adjacent, and in contact with, the second surface 221b of the wound contact layer, and the second surface 222b of the transmission layer 222 is adjacent, and in contact with, the first surface 223a of the superabsorbent layer 223.

The superabsorbent layer 223 may comprise a first surface 223a and a second surface 223b. In use, the first surface 223a may be inwardly facing toward the patient's body, and the second surface 223b may be outwardly facing away from the patient's body. The first surface 223a of the superabsorbent layer 223 may be adjacent, and in contact with, the second surface 221b of the wound contact layer 221 or the second surface 222b of the transmission layer 222. The second surface 223b of the superabsorbent layer 223 may be adjacent, and in contact with, the first surface 222a of the transmission layer 222 or the first surface 230c of the backing layer 230. In the described embodiment, the first surface 223a of the superabsorbent layer 223 is adjacent, and in contact with, the second surface 222b of the transmission layer 222, and the second surface 223b of the superabsorbent layer 223 is adjacent, and in contact with, the first surface 230c of the backing layer 230.

The adhesive skin contact layer 220 may radially overlap the absorbent pad 210. The adhesive skin contact layer 220 may comprise an interior portion 220' adjacent to and surrounding the window 220a, and an exterior portion 220" radially outwardly from the interior portion. The interior portion and the exterior portion of the first surface 220c of the adhesive skin contact layer 220 may contact and adhere to the skin of a patient in use. The interior portion of the second surface 220d of the adhesive skin contact layer 220 may be adjacent, and in contact with, a portion of the first surface 221a of the wound contact layer 221. The exterior portion of the second surface 220b of the adhesive skin contact layer 220 may be adjacent, and joined with, a peripheral portion of the first surface 230c of the backing layer 230. Beneficially, this helps to prevent wound exudate escaping from the confines of the wound dressing which would be unhygienic and cause discomfort for the user. Moreover, this also helps maintain a negative pressure at the wound site. Furthermore, it maintains the structure of the dressing prior to use, meaning the dressing is integrally formed as a "one-piece" dressing.

The exterior portion of the second surface 220d of the adhesive skin contact layer 220 may be joined to the peripheral portion of the first surface 230c of the backing layer 230 by heat lamination, adhesive, welding or stitching.

The exterior portion of the second surface 220d of the adhesive skin contact layer 220 may extend radially beyond the periphery of the absorbent structure by about 12 mm.

A central region 202 of the dressing 201 may generally refer to the area in which a wound would be located when in use, i.e. within a region of the wound contact layer 221. In some embodiments the central region of the dressing may refer to a circular (or elliptical) region arranged at the interface between the first 210 and second 211 elongate portions of the absorbent pad 210 and encompassing some or all of the wings 212,213. It will be appreciated however that the layers of the wound dressing in the central region 202 does not have a different composition to the remainder of the same layer.

The provision of wings 212,213 greatly increases the size of the central region 2, as can be seen in Figure 7, a circular central region 202' bounded by the corners of the first and second elongate members has an area substantially smaller than a circular central region 202 bound by the wings 212,213. Indeed, in the present embodiment, the wings 212,213 represent a modest 6% increase in surface area of the absorbent pad 210, however they provide an approximately 40% increase in the area of the central region. This allows the wound dressing 201 to be used on larger wounds and/or reduces the accuracy with which the dressing needs to be applied to the wound site.

The wound dressing 201 is shaped to conform more readily to different areas of a patient's body which are typically but not necessarily contoured and/or difficult areas to adhere a dressing to. For example, these areas may include a shoulder, armpit, elbow, wrist, sacral region, hip and in particular the breast and heel.

In this embodiment the wound dressing 201 has a length (as measured between two opposing peripheral edges of the covering layer) of 220 mm, and a width (as measured between the other two opposing peripheral edges of the covering layer) of 220 mm.

In this embodiment the first elongate portion has a length of 100 mm and a width of 60 mm, and the second elongate portion has a length of 160 mm and a width of 60 mm therefor, the length of the absorbent pad 210, which comprises the length of the first elongate portion and the width of the second elongate portion is 160 mm. The maximum width of the absorbent pad 210 is 180 mm, as measured along the length of the second elongate portion. The concavely curved edge 213a,214a of each wing 213,214 has a radius of curvature of 30 mm.

The convex corners of the absorbent pad 210, that is the two corners of the first elongate portion 211 distal to the wings 213,214 and the four corners of the second elongate portion 212 are rounded with a radius of 10 mm. The notches 240a,b on the second elongate portion 212 have a depth of 10 mm and a width of 40 mm, as such in the present embodiment, the notches extend the entire distance between the rounded corners of their respective ends of the second elongate portion 212.

The absorbent pad 210 is arranged centrally within the wound dressing 201, due to the different shapes of the absorbent pad 210 the covering layer 230 the width of the border region 233 (as measured perpendicular from the periphery of the absorbent pad 10 to the peripheral edge 231 of the covering layer 230) is not uniform. Minima in the width of the border region 233 are found adjacent to a number of points; at a second short edge of the first elongate portion 211b and second long edge 212b of the second elongate portion 212 the width of the border region is 30 mm, and at the two short edges 212c,d of the second elongate portion 212 the width of the border region is 20 mm.

The interior region 220' of the adhesive skin contact layer 220 has a width of 12 mm, this provides a sufficient degree of retention of the absorbent pad 212 without hindering the effectiveness thereof.

Of course, the aforementioned teachings could be applied to dressings of other sizes. For example, the dressing could be uniformly scaled down for use with smaller wound sites, or uniformly scaled up for larger wound sites. Equally, the dressing may be non-uniformly scaled, thereby providing a more elongate dressing or a wider dressing as required to suit a particular wound site whilst still retaining the overall T-shaped outline of the absorbent pad 210. Likewise, the width of the border region could be varied, for example from between 10 mm and 50 mm with a wider border region providing greater adhesion to the wound site and an improved seal for the wound dressing cavity.

In use the wound dressing 201 is applied to the wound site such that the central region 202 is arranged over the wound site and is secured in place using the adhesive skin contact layer 220. The central region 202 is applied over the wound with the distal ends of the second elongate portion 212c,d, the wings 213,214 help the wound dressing 201 conform when folded or bent over a contour and also provide an increased volume of absorbent pad 210 proximate to the wound, this provides improved fluid management at the wound site. The notches 240 further improve the ability of the dressing to adapt to the contours of the wound site, providing a preferential position for the dressing 201 to fold at and reducing the possibility for bulging. The shape of the wound dressing 201 lends itself to being folded as described above. When folded the border region 233, in particular the portion outward of the wings 213,214 may bulge, due to the increased amount of covering layer 230 in the portion, a HCP can readily cut this part to assist in conforming the dressing to the wound site without risk of compromising the seal, indeed the large amount of material lends itself to being overlapped once cut, thereby improving the seal. This effect is particularly pronounced in a preferred embodiment where the covering layer 230 and adhesive skin contact layer 220 comprises flexible film adhesive constructs (for example a polyurethane film with a silicone adhesive), which are easily cut and respond well to being overlapped.

Next the wound dressing 1 to a source of non-atmospheric pressure (for example, when configured for use in NPWT, a pump) via an airway 137. To further improve the seal sealing strips (not shown) may applied to the perimeter of the dressing 201 to ensure that there are no leaks. The provision of the aperture 235 over the first elongate portion 211 of the absorbent pad 210 is particularly advantageous as it is beneficial to the user that the connection to the non-atmospheric pressure (e.g. airway) is not arranged above the wound site. In instances where the port is provided above the wound site an external application of pressure onto the dressing, for example where the user lies on it or rests against it, applies greater pressure at the port due to it raised nature.

In other instances, in particular where the wound is linear, for example as a result of an incision, the wound dressing 201 can be arranged such that the second elongate portion 212 extends along the wound, in such instances the wings 213,214 again provide an increase in absorbent pad volume proximate to the wound site.

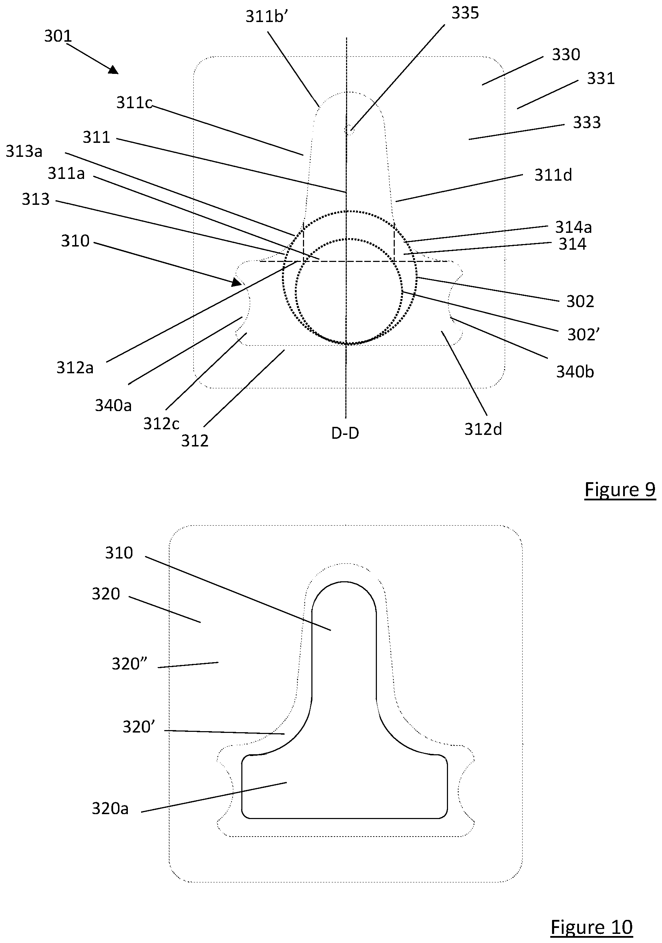

Figures 9 to 12 show a wound dressing 301 according to a fourth embodiment of the present disclosure. The dressings are considered to be favourable for attaching or adhering to multiple different sites on the human body in particular to the heel or breast.

The wound dressing 301 of the fourth embodiment bears a number of similarities to the dressing of the first 1, second 101 and third 201 embodiments, with like features having the same numerals, advanced by 300, 200 and 100 respectively. As compared to the preceding embodiments the wound dressing 301 of the fourth embodiment combines the features of the second and third embodiments.

Figure 10 shows the wound dressing 301 in plan. The dressing 301 comprises an absorbent pad 310. To aid the description of the dressing 301 shape, there is shown a centre line **D-D** which extends centrally along the major axis of the dressing. The length of the dressing 301 is measured along the major axis and the width of the dressing 310, is measured perpendicular to the major axis.

The absorbent pad 310 has an overall "T-shape", that is it comprises a first elongate portion 311, and a second elongate portion 312. A short edge 311a of the first elongate portion 311 abuts the second elongate portion 312 perpendicularly at a midpoint of a first long edge 312a.

Extending between a first long edge 311c of the first elongate portion 311 and the long edge 312a of the second elongate portion 312 there is provided a first wing 313. The first wing 313 has a triangular shape, with two short edges abutting the first long edge 311c of the first elongate portion 311 and the long edge 312a of the second elongate portion 312. The third edge 313a has a concavely curving profile.

Likewise, extending between a second long edge 311d of the first elongate portion 311 and the long edge 312a of the second elongate portion 312 there is provided a second wing 314. The first wing 313 has a triangular shape, with two short edges abutting the second long edge 311d of the first elongate portion 311 and the long edge 312a of the second elongate portion 312. The third edge 414a has a concavely curving profile.

To improve the ability of the dressing for conform to the contours of the wound site, the short edges 312c,d are provided with a notch 340a,b. Each notch 340a,b has a curved profile with a depth one quarter the width of the notch.

The dressing 301 will typically be places such that the second elongate portion 312 of the absorbent pad, or the region at the interface between the first 311 and second 312 elongate portions is arranged over the wound. The end of the first elongate portion 311 distal to the second elongate portion 312 plays a reduced role in extrudate management, as such it is beneficial to reduce the volume of this area thereby improving the efficiency of the delivery of non-atmospheric pressure. To this end the first elongate portion 311 tapers the width decreasing away from the interface with the second elongate portion 312 and the distal end 331b' is rounded.

Arranged above and extending beyond a periphery of the absorbent pad 310 is a covering layer 330, the covering layer is provided with a peripheral edge 331, the peripheral edge 331 has a square shape with rounded corners. The covering layer 330 and wound may define a wound dressing cavity in which the remaining layers of the dressing 301 are provided. Thus, the covering layer 330 defines the shape of the dressing. The region of the covering layer 330 between the peripheral edge 331 and the periphery of the absorbent pad may be referred to as a border region 333.

The covering layer 330 of the dressing is provided as a bacterial and viral barrier layer which preferably resists the ingress of liquid and air but allows moisture vapour transmission. In this way the covering layer 330 enhances the overall fluid handling capacity of the dressing by allowing for the escape of moisture vapour through the cover while enabling the application of pressure (either positive or negative) to the wound. The outer cover layer is for instance a layer having a MVTR of at least 3,000 g m⁻² per 24 hours or, more preferably, in the range of from 10,000gm⁻² to 50,000g m⁻² per 24 hours measured by the method described in BS EN 13726-2 2002 "Test methods for primary wound dressings Part 2 Moisture vapour transmission rate of permeable film dressings". The covering layer may be any suitable material and may, in some embodiments, comprise a polyurethane material, as known in the art. The covering layer may have a thickness of between 25 microns and 35 microns, preferably 30 microns.

To provide fluid communication between the wound dressing cavity and a source of non-atmospheric pressure, the covering layer 330 is provided with an aperture 335. The aperture 335 is arranged over a centre line of the first elongated portion 311 of the absorbent pad 310 approximately 75% of the length of the first elongate portion 311 distal from the interface between the first 311 and second 312 elongate portions.

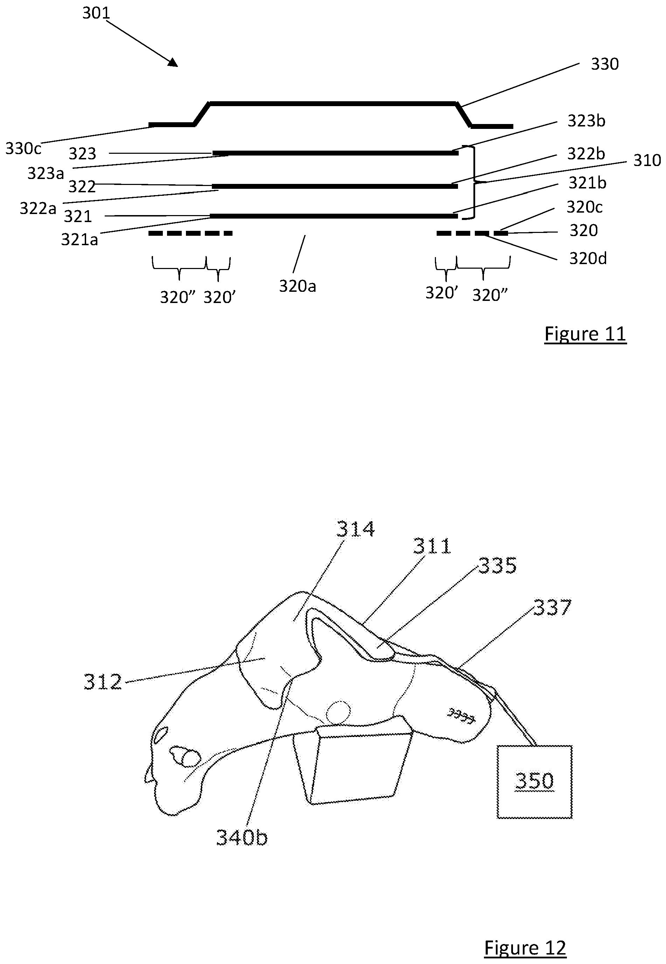

As shown in figure 11, the absorbent pad may be comprised of a plurality of layers. In order from the wound (or peri-wound area) to the covering layer 330 these may be an adhesive skin contact layer 320, a wound contact layer 321, a transmission layer 322, and a superabsorbent layer 323.

In some embodiments the wound contact layer 321, the transmission layer 322, and the superabsorbent layer 323 may be laminated together, this means each of the layers form a consolidated, absorbent and retentive structure. Advantageously, this reduces or removed the need for additional components to maintain the structure, thereby reducing the bulk of the dressing and improving flexibility and further enhancing the ability of the dressing to conform to contoured wound sites.

As shown in Figure 10, the adhesive skin contact layer 320 may have a perimetral shape with a central window 320a, the outer periphery 320b of the adhesive skin contact layer may be contiguous with the peripheral edge 331 of the covering layer 330. The window 320a is a through hole in the adhesive skin contact layer, it is the same shape as the absorbent pad, but is sized such that it is smaller than the absorbent pad 310. As such the adhesive skin contact layer 320 retains the absorbent pad 310 in position between the adhesive skin contact layer 320 and the covering layer 330.

The adhesive skin contact layer 320 comprises an upper surface 320c and a lower surface 320d. The lower surface 320d contacts the dermal surface in use. In use, the lower surface 320d of the adhesive skin contact layer 320 is detachably adhered to a dermal surface (not shown) of a patient such that the wound site (not shown) is located within the window 320a. As such, the adhesive skin contact layer 320 is not in direct contact with the wound site but does wholly surround it. Thus, the adhesive skin contact layer 320 is in contact with intact peri-wound skin only. The upper surface 320b may be outwardly facing away from the patient's body.

A removable cover or "release layer" (not shown) may be adhered to the lower surface 320d of the adhesive skin contact layer 320. The removable cover, which may comprise folded grip sections, is removable from the lower surface 20d of the adhesive skin contact layer 320. Thus, the removable cover protects the adhesive skin contact layer 320 when the removable cover is adhered to the adhesive skin contact layer 320, but when the removable cover is removed from the lower surface 320d of the adhesive skin contact layer 320 for use of the wound dressing 301, the lower surface 320d is exposed and able to releasably secure the wound dressing 301 to the skin of a patient.

The wound contact layer 321 may comprise a first surface 321a and a second surface 321b. In use, the first surface 321a may be inwardly facing toward the patient's body, and the second surface 321b may be outwardly facing away from the patient's body. The first surface 321a of the wound contact layer 321 may contact the wound when the wound dressing 301 is adhered to the skin adjacent to the wound (i.e., when in use). The first surface 321a of the wound contact layer 304 is substantially aligned with the window 320a of the adhesive skin contact layer 320. As such, in use, the first surface 321a contacts the wound through the window 320a. The second surface 321b of the wound contact layer 321 may be adjacent, and in contact with, a first surface 322a of the transmission layer 322 or a first surface 323a of the superabsorbent layer 323. In the described embodiment, the second surface 322b of the wound contact layer 322 is adjacent, and in contact with, the first surface 323a of the transmission layer 323.

The transmission layer 322 may comprise a first surface 322a and a second surface 322b. In use, the first surface 322a may be inwardly facing toward the patient's body, and the second surface 322b may be outwardly facing away from the patient's body. The first surface 322a of the transmission layer 322 may be adjacent, and in contact with, the second surface 321b of the wound contact layer 321 or a second surface 323b of the superabsorbent layer 323. The second surface 322b of the transmission layer 322 may be adjacent, and in contact with, a first surface 323a of the superabsorbent layer 323 or the first surface 330c of the covering layer 330. In the described embodiment, the first surface 322a of the transmission layer 322 is adjacent, and in contact with, the second surface 321b of the wound contact layer, and the second surface 322b of the transmission layer 322 is adjacent, and in contact with, the first surface 323a of the superabsorbent layer 323.

The superabsorbent layer 323 may comprise a first surface 323a and a second surface 323b. In use, the first surface 323a may be inwardly facing toward the patient's body, and the second surface 323b may be outwardly facing away from the patient's body. The first surface 323a of the superabsorbent layer 323 may be adjacent, and in contact with, the second surface 321b of the wound contact layer 321 or the second surface 322b of the transmission layer 322. The second surface 323b of the superabsorbent layer 323 may be adjacent, and in contact with, the first surface 322a of the transmission layer 322 or the first surface 330c of the backing layer 330. In the described embodiment, the first surface 323a of the superabsorbent layer 323 is adjacent, and in contact with, the second surface 322b of the transmission layer 322, and the second surface 323b of the superabsorbent layer 323 is adjacent, and in contact with, the first surface 330c of the backing layer 330.

The adhesive skin contact layer 320 may radially overlap the absorbent pad 310. The adhesive skin contact layer 320 may comprise an interior portion 320' adjacent to and surrounding the window 320a, and an exterior portion 320" radially outwardly from the interior portion. The interior portion and the exterior portion of the first surface 20c of the adhesive skin contact layer 320 may contact and adhere to the skin of a patient in use. The interior portion of the second surface 320d of the adhesive skin contact layer 320 may be adjacent, and in contact with, a portion of the first surface 321a of the wound contact layer 321. The exterior portion of the second surface 320b of the adhesive skin contact layer 320 may be adjacent, and joined with, a peripheral portion of the first surface 330c of the backing layer 330. Beneficially, this helps to prevent wound exudate escaping from the confines of the wound dressing which would be unhygienic and cause discomfort for the user. Moreover, this also helps maintain a negative pressure at the wound site. Furthermore, it maintains the structure of the dressing prior to use, meaning the dressing is integrally formed as a "one-piece" dressing.

The exterior portion of the second surface 320d of the adhesive skin contact layer 320 may be joined to the peripheral portion of the first surface 330c of the backing layer 330 by heat lamination, adhesive, welding or stitching.

The exterior portion of the second surface 320d of the adhesive skin contact layer 320 may extend radially beyond the periphery of the absorbent structure by about 12 mm.

A central region 302 of the dressing 301 may generally refer to the area in which a wound would be located when in use, i.e. within a region of the wound contact layer 321. In some embodiments the central region of the dressing may refer to a circular (or elliptical) region arranged at the interface between the first 311 and second 312 elongate portions of the absorbent pad 310 and encompassing some or all of the wings 313,314. It will be appreciated however that the layers of the wound dressing in the central region 302 does not have a different composition to the remainder of the same layer.

The provision of wings 312,313 greatly increases the size of the central region 302, as can be seen in Figure 9, a circular central region 302' bounded by the corners of the first and second elongate members has an area substantially smaller than a circular central region 302 bound by the wings 312,313. Indeed, in the present embodiment, the wings 312,313 represent a modest 5% increase in surface area of the absorbent pad 310, however they provide an approximately 40% increase in the area of the central region. This allows the wound dressing 301 to be used on larger wounds and/or reduces the accuracy with which the dressing needs to be applied to the wound site.

The wound dressing 301 is shaped to conform more readily to different areas of a patient's body which are typically but not necessarily contoured and/or difficult areas to adhere a dressing to. For example, these areas may include a shoulder, armpit, elbow, wrist, sacral region, hip and in particular the breast and heel.

In this embodiment the wound dressing 301 has a length (as measured along D-D between two opposing peripheral edges of the covering layer 330) of 235 mm, and a width (as measured between the other two opposing peripheral edges of the covering layer 330) of 220 mm.

In this embodiment the first elongate portion 311 has a length of 120 mm and a width tapering from 60 mm proximate to the wings 113,114 to 50 mm proximate to the aperture 35. The second elongate portion 312 has a length of 160 mm and a width of 60 mm. Therefore, the length of the absorbent pad 310, which comprises the length of the first elongate portion 311 and the width of the second elongate portion 312 is 180 mm. The maximum width of the absorbent pad 310 is 160 mm, as measured along the length of the second elongate portion 312. The concavely curved edge 313a,314a of each wing 313,314 has a radius of curvature of 40 mm.

The distal end 311c of the first elongate portion 311 of the absorbent pad 310 is rounded with a radius of 25 mm and thus the distal end 311c terminates as a semicircular profile, this further reduces the volume of the first elongate portion. The convex corners of the second elongate portion are rounded with a radius of 10 mm.

The convex corners of the absorbent pad 310, that is the two corners of the first elongate portion 311 distal to the wings 312,314 and the four corners of the second elongate portion are rounded with a radius of 10 mm.

The notches 340a,b on the second elongate portion 312 have a depth of 10 mm and a width of 40 mm, as such, in the present embodiment, the notches extend the entire distance between the rounded corners of their respective ends of the second elongate portion 312.

The absorbent pad 310 is arranged centrally within the wound dressing 301, due to the different shapes of the absorbent pad 310 the covering layer 330 the width of the border region 333 (as measured perpendicular from the periphery of the absorbent pad 310 to the peripheral edge 331 of the covering layer 330) is not uniform. Minima in the width of the border region 333 are found adjacent to a number of points; at the rounded distal end 311c of the first elongate portion 311 the border region has a width of 25 mm, at the two short edges 312c,d and second long edge 312b of the second elongate portion 312 the width of the border region is 30 mm.

The interior region 320' of the adhesive skin contact layer 320 was a width of 12 mm, this provides a sufficient degree of retention of the absorbent pad 312 without hindering the effectiveness thereof.

Of course, the aforementioned teachings could be applied to dressings of other sizes. For example, the dressing could be uniformly scaled down for use with smaller wound sites, or uniformly scaled up for larger wound sites. Equally, the dressing may be non-uniformly scaled, thereby providing a more elongate dressing or a wider dressing as required to suit a particular wound site whilst still retaining the overall T-shaped outline of the absorbent pad 310. Likewise, the width of the border region could be varied, for example from between 10 mm and 50 mm with a wider border region providing greater adhesion to the wound site and an improved seal for the wound dressing cavity.

With reference to Figure 12, in use the wound dressing 301 is applied to the wound site such that the central region 302 is arranged over the wound site and is secured in place using the adhesive skin contact layer 320. In the present example the dressing 301 is applied to a patient with a wound on their heel. The central region 302 is applied over the wound with the distal ends of the second elongate portion 312c,d being folded onto the sides of the foot, the wings 313,314 help the wound dressing 301 conform to this fold and also provide an increased volume of absorbent pad 310 proximate to the wound, this provides improved fluid management at the wound site. The notches 340 further improve the ability of the dressing to adapt to the contours of the wound site, providing a preferential position for the dressing 301 to fold at and reducing the possibility for bulging. This effect is particularly pronounced in a preferred embodiment where the covering layer 330 and adhesive skin contact layer 320 comprises flexible film adhesive constructs (for example a polyurethane film with a silicone adhesive), which are easily cut and respond well to being overlapped.

The first elongate region 311 is folded around the heel and extends up the back of the ankle. The shape of the wound dressing 301 lends itself to being folded as described above. When folded the board region 333, in particular the portion outward of the wings 313,314 may bulge, due to the increased amount of covering layer 330 in the portion, a HCP can readily cut this part to assist in conforming the dressing to the wound site without risk of compromising the seal, indeed the large amount of material lends itself to being overlapped once cut, thereby improving the seal.

Next a source of non-atmospheric pressure 350 (for example, when configured for use in NPWT, a pump) is attached to the wound dressing via an airway 337 attached to the aperture 335. To further improve the seal sealing strips (not shown) may applied to the perimeter of the dressing 301 to ensure that there are no leaks. The provision of the aperture 335 over the first elongate portion 311 of the absorbent pad 310 is particularly advantageous as it is beneficial to the user that the connection to the non-atmospheric pressure (e.g. airway 337) is not arranged above the wound site. In instances where the port is provided above the wound site an external application of pressure onto the dressing, for example where the user lies on it or rests against it, applies greater pressure at the port due to it raised nature.

In other instances, in particular where the wound is linear, for example as a result of an incision, the wound dressing 301 can be arranged such that the second elongate portion 312 extends along the wound, in such instances the wings again provide an increase in absorbent pad volume proximate to the wound site.

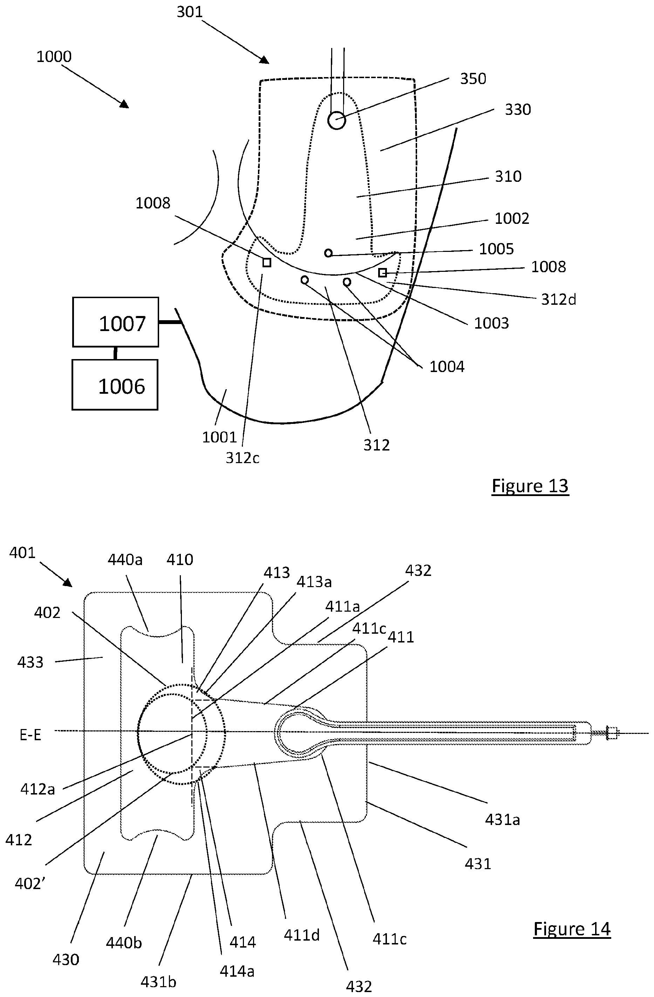

With reference to Figure 13 a trial of the wound dressing 301 of the fourth embodiment will be described. A test apparatus 1000 comprising a model female torso 1001 with breast 1002 is provided. Arranged on the torso 1001 adjacent to a lower edge 1003 of the breast 1002 are two fluid inlet ports 1004, a further fluid inlet port 1005 is provided on a lower portion of the breast 1002. These fluid inlet ports 1004,1005 simulate a typical anchor shaped incision on the breast. The fluid inlet ports 1004,1005 have are circular with a diameter of 1 mm and are fluidly connected to a fluid reservoir 1006 and pump 1007. The reservoir 1006 contains a test fluid which has an ionic composition comparable to wound exudate, the fluid is dyed to help visualise the spread of the fluid within the wound dressing 301. The pump 1007 is configured to supply a total of 1.1 ml/hr/cm² to the fluid inlet ports to simulate exudate from an incision.

The test apparatus further comprises two pressure sensors 1008 on the torso 1001 just below the breast 1002. As will be expanded upon below they are positioned so that they are covered by the distal ends 312c,d of the second elongate portion of the absorbent pad 310 of the dressing 301.

The apparatus 1000 is designed to simulate an incision just below the breast. For the trail a dressing 301 is placed on the apparatus to cover fluid inlet ports 1004,1005, the second elongate portion 312 extending along the lower edge of the breast and the first elongate portion 311 up the breast. The covering layer 330 is sealed against the torso.

As in the exemplary use described above in reference to Figure 12, a negative pressure supply is connected to the dressing via the aperture 350. Once the dressing was applied the pumps supply 1.1ml/hr/cm² of test fluid. The negative pressure (in mmHg) was then recorded by the pressure sensors for 72 hours at 10s intervals.

The pressure as measured at the pressure sensors 1008 remained consistent throughout the test at a nominal pressure of -80 mmHg, with the two sensors being in good agreement throughout.

This indicates that the dressing 301 was able to transmit negative pressure from the aperture to distal ends 312c,d of the second elongate portion 312 over the exudate.

Figures 14 to 16 show a wound dressing 401 according to a fifth embodiment of the present disclosure. The dressings are considered to be favourable for attaching or adhering to multiple different sites on the human body in particular to the heel or breast.

The wound dressing 401 of the fifth embodiment bears a number of similarities to the dressing of the first 1, second 101, third 201 embodiments and in particular the fourth 301 embodiment, with like features having the same numerals, advanced by 400, 300, 200 and 100 respectively.

Figure 13 shows the wound dressing 401 in plan. The dressing 401 comprises an absorbent pad 410. To aid the description of the dressing 401 shape, there is shown a centre line E-E which extends centrally along the major axis of the dressing. The length of the dressing 401 is measured along the major axis and the width of the dressing 410, is measured perpendicular to the major axis.

The absorbent pad 410 has an overall "T-shape", that is it comprises a first elongate portion 411, and a second elongate portion 412. A short edge 411a of the first elongate portion 411 abuts the second elongate portion 412 perpendicularly at a midpoint of a first long edge 412a.

Extending between a first long edge 411c of the first elongate portion 411 and the long edge 412a of the second elongate portion 412 there is provided a first wing 413. The first wing 413 has a triangular shape, with two short edges abutting the first long edge 411c of the first elongate portion 411 and the long edge 412a of the second elongate portion 412. The third edge 413a has a concavely curving profile.

Likewise, extending between a second long edge 411d of the first elongate portion 411 and the long edge 412a of the second elongate portion 412 there is provided a second wing 414. The second wing 413 has a triangular shape, with two short edges abutting the second long edge 411d of the first elongate portion 411 and the long edge 412a of the second elongate portion 412. The third edge 414a has a concavely curving profile.

To improve the ability of the dressing to conform to the contours of the wound site, the short edges 412c,d of the second elongate portion are provided with a notch 440a,b. Each notch 440a,b has a curved profile with a depth one quarter the width of the notch.

The dressing 401 will typically be placed such that the second elongate portion 412 of the absorbent pad, or the region at the interface between the first 411 and second 412 elongate portions is arranged over the wound. The end of the first elongate portion 411 distal to the second elongate portion 412 plays a reduced role in extrudate management, as such it is beneficial to reduce the volume of this area thereby improving the efficiency of the delivery of non-atmospheric pressure. To this end the first elongate portion 411 tapers the width decreasing away from the interface with the second elongate portion 412 and the distal end 411c is rounded.

Arranged above and extending beyond a periphery of the absorbent pad 410 is a covering layer 430, the covering layer is provided with a peripheral edge 431, the peripheral edge 431 has a "T-shaped" shape with rounded corners. The "T-shape" is defined by a covering layer notch 432 on two corners of an otherwise square covering layer (for example as found in the fourth embodiment, which has a similarly shaped absorbent pad). The two notches 432 are provided on the two corners between the peripheral edge 431a of the covering layer 430 perpendicular to the centre line E-E and proximal to the first elongate portion 411 and the two edges parallel 431b to the centre line E-E. The notches 432 each extend 1/6 of the width of the covering layer 430 in from the parallel edges 431b and 1/3 the length of the covering layer 430 in from the perpendicular edge 431a.

The covering layer 430 and wound may define a wound dressing cavity in which the remaining layers of the dressing 401 are provided. Thus, the covering layer 430 defines the overall shape of the dressing 401. The region of the covering layer 430 between the peripheral edge 431 and the periphery of the absorbent pad may be referred to as a border region 433.

The covering layer 430 of the dressing is provided as a bacterial and viral barrier layer which preferably resists the ingress of liquid and air but allows moisture vapour transmission. In this way the covering layer 430 enhances the overall fluid handling capacity of the dressing by allowing for the escape of moisture vapour through the cover while enabling the application of pressure (either positive or negative) to the wound. The outer cover layer is for instance a layer having a MVTR of at least 3,000 g m⁻² per 24 hours or, more preferably, in the range of from 10,000gm⁻² to 50,000g m⁻² per 24 hours measured by the method described in BS EN 13726-2 2002 "Test methods for primary wound dressings Part 2 Moisture vapour transmission rate of permeable film dressings". The covering layer may be any suitable material and may, in some embodiments, comprise a polyurethane material, as known in the art. The covering layer may have a thickness of between 25 microns and 35 microns, preferably 30 microns.

To provide fluid communication between the wound dressing cavity and a source of non-atmospheric pressure, the covering layer 430 is provided with an aperture 435. The aperture 435 is arranged over a centre line of the first elongated portion 411 of the absorbent pad 410 approximately 80% of the length of the first elongate portion 411 distal from the interface between the first 411 and second 412 elongate portions.

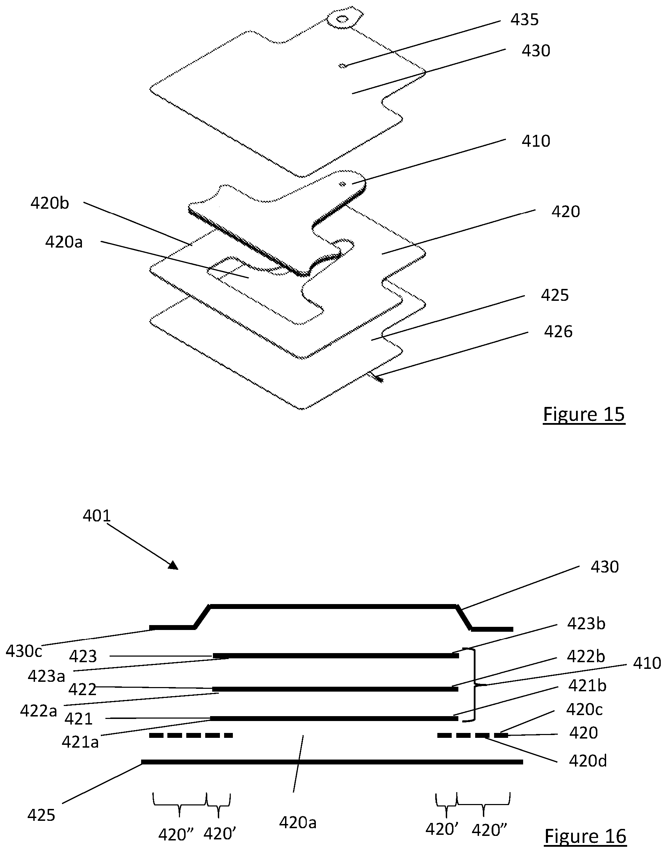

As shown in Figures 14 and 15, the absorbent pad may be comprised of a plurality of layers. In order from the wound (or peri-wound area) to the covering layer 430 these may be an adhesive skin contact layer, a wound contact layer 421, a transmission layer 422, and a superabsorbent layer 423.

In some embodiments the absorbent pad 410 comprises the wound contact layer 421, the transmission layer 422, and the superabsorbent layer 423 which may be laminated together, this means each of the layers form a consolidated, absorbent and retentive structure. Advantageously, this reduces or removed the need for additional components to maintain the structure, thereby reducing the bulk of the dressing and improving flexibility and further enhancing the ability of the dressing to conform to contoured wound sites.

As shown in Figure 15, the adhesive skin contact layer 420 may have a perimetral shape with a central window 420a, the outer periphery 420b of the adhesive skin contact layer may be contiguous with the peripheral edge 431 of the covering layer 430. The window 420a is a through hole in the adhesive skin contact layer, it is the same shape as the absorbent pad 410, but is sized such that it is smaller than the absorbent pad 410. As such the adhesive skin contact layer 420 retains the absorbent pad 410 in position between the adhesive skin contact layer 420 and the covering layer 430.

As shown in Figure 16, which is a cross-sectional view of the dressing 401 along the centre line E-E with the layers separated for ease of viewing, the adhesive skin contact layer 420 comprises an upper surface 420c and a lower surface 420d. The lower surface 420d contacts the dermal surface in use. In use, the lower surface 420d of the adhesive skin contact layer 420 is detachably adhered to a dermal surface (not shown) of a patient such that the wound site (not shown) is located within the window 420a. As such, the adhesive skin contact layer 420 is not in direct contact with the wound site but does wholly surround it. Thus, the adhesive skin contact layer 420 is in contact with intact peri-wound skin only. The upper surface 420b may be outwardly facing away from the patient's body.

A removable cover or "release layer" 425 may be adhered to the lower surface 420d of the adhesive skin contact layer 420. The removable cover 425, which may comprise folded grip sections 426, is removable from the lower surface 420d of the adhesive skin contact layer 420. Thus, the removable cover protects the adhesive skin contact layer 420 when the removable cover is adhered to the adhesive skin contact layer 420, but when the removable cover is removed from the lower surface 420d of the adhesive skin contact layer 420 for use of the wound dressing 401, the lower surface 320d is exposed and able to releasably secure the wound dressing 401 to the skin of a patient.

The wound contact layer 421 may comprise a first surface 421a and a second surface 421b. In use, the first surface 421a may be inwardly facing toward the patient's body, and the second surface 421b may be outwardly facing away from the patient's body. The first surface 421a of the wound contact layer 421 may contact the wound when the wound dressing 401 is adhered to the skin adjacent to the wound (i.e., when in use). The first surface 421a of the wound contact layer 404 is substantially aligned with the window 420a of the adhesive skin contact layer 420. As such, in use, the first surface 421a contacts the wound through the window 420a. The second surface 421b of the wound contact layer 421 may be adjacent, and in contact with, a first surface 422a of the transmission layer 422 or a first surface 423a of the superabsorbent layer 423. In the described embodiment, the second surface 422b of the wound contact layer 422 is adjacent, and in contact with, the first surface 423a of the transmission layer 423.

The transmission layer 422 may comprise a first surface 422a and a second surface 422b. In use, the first surface 422a may be inwardly facing toward the patient's body, and the second surface 422b may be outwardly facing away from the patient's body. The first surface 422a of the transmission layer 422 may be adjacent, and in contact with, the second surface 421b of the wound contact layer 421 or a second surface 423b of the superabsorbent layer 423. The second surface 422b of the transmission layer 422 may be adjacent, and in contact with, a first surface 423a of the superabsorbent layer 423 or the first surface 430c of the covering layer 430. In the described embodiment, the first surface 422a of the transmission layer 422 is adjacent, and in contact with, the second surface 421b of the wound contact layer, and the second surface 422b of the transmission layer 422 is adjacent, and in contact with, the first surface 423a of the superabsorbent layer 423.

The superabsorbent layer 423 may comprise a first surface 423a and a second surface 423b. In use, the first surface 423a may be inwardly facing toward the patient's body, and the second surface 423b may be outwardly facing away from the patient's body. The first surface 423a of the superabsorbent layer 423 may be adjacent, and in contact with, the second surface 421b of the wound contact layer 421 or the second surface 422b of the transmission layer 422. The second surface 423b of the superabsorbent layer 423 may be adjacent, and in contact with, the first surface 422a of the transmission layer 422 or the first surface 430c of the backing layer 430. In the described embodiment, the first surface 423a of the superabsorbent layer 423 is adjacent, and in contact with, the second surface 422b of the transmission layer 422, and the second surface 423b of the superabsorbent layer 423 is adjacent, and in contact with, the first surface 430c of the backing layer 430.

The adhesive skin contact layer 420 may radially overlap the absorbent pad 410. The adhesive skin contact layer 420 may comprise an interior portion 420' adjacent to and surrounding the window 420a, and an exterior portion 420" radially outwardly from the interior portion. The interior portion and the exterior portion of the first surface 420c of the adhesive skin contact layer 420 may contact and adhere to the skin of a patient in use. The interior portion of the second surface 420d of the adhesive skin contact layer 420 may be adjacent, and in contact with, a portion of the first surface 421a of the wound contact layer 421. The exterior portion of the second surface 420b of the adhesive skin contact layer 420 may be adjacent, and joined with, a peripheral portion of the first surface 430c of the backing layer 430. Beneficially, this helps to prevent wound exudate escaping from the confines of the wound dressing 401 which would be unhygienic and cause discomfort for the user. Moreover, this also helps maintain a negative pressure at the wound site. Furthermore, it maintains the structure of the dressing prior to use, meaning the dressing is integrally formed as a "one-piece" dressing.

The exterior portion of the second surface 420d of the adhesive skin contact layer 420 may be joined to the peripheral portion of the first surface 430c of the backing layer 430 by heat lamination, adhesive, welding or stitching.

The exterior portion of the second surface 420d of the adhesive skin contact layer 420 may extend radially beyond the periphery of the absorbent structure by about 12 mm.

A central region 402 of the dressing 401 may generally refer to the area in which a wound would be located when in use, i.e. within a region of the wound contact layer 421. In some embodiments the central region of the dressing may refer to a circular (or elliptical) region arranged at the interface between the first 411 and second 412 elongate portions of the absorbent pad 410 and encompassing some or all of the wings 413,414. It will be appreciated however that the layers of the wound dressing in the central region 402 does not have a different composition to the remainder of the same layer.

The provision of wings 412,413 greatly increases the size of the central region 402, as can be seen in Figure 13, a circular central region 402' bounded by the corners of the first and second elongate members has an area substantially smaller than a circular central region 402 bound by the wings 412,413. Indeed, in the present embodiment, the wings 412,413 represent a modest 5% increase in surface area of the absorbent pad 410, however they provide an approximately 40% increase in the area of the central region. This allows the wound dressing 401 to be used on larger wounds and/or reduces the accuracy with which the dressing needs to be applied to the wound site.

The wound dressing 401 is shaped to conform more readily to different areas of a patient's body which are typically, but not necessarily, contoured and/or difficult areas to adhere a dressing to. For example, these areas may include a shoulder, armpit, elbow, wrist, sacral region, hip and in particular the breast and heel.

In this embodiment the wound dressing 401 has a length (as measured along E-E between two opposing peripheral edges of the covering layer 430) of 270 mm, and a width (as measured between the other two opposing peripheral edges of the covering layer 330) of 270 mm.

In this embodiment the first elongate portion 411 has a length of 130 mm and a width tapering from 60 mm proximate to the wings 413,414 to 50 mm proximate to the aperture 435. The second elongate portion 412 has a length of 205 mm and a width of 70 mm. Therefore, the length of the absorbent pad 410, which comprises the length of the first elongate portion 411 and the width of the second elongate portion 412 is 200 mm. The maximum width of the absorbent pad 410 is 2050 mm, as measured along the length of the second elongate portion 412. The concavely curved edge 413a,414a of each wing 413,414 has a radius of curvature of 25 mm.

The distal end 411c of the first elongate portion 411 of the absorbent pad 410 is rounded with a radius of 25 mm and thus the distal end 411c terminates as a semicircular profile, this further reduces the volume of the first elongate portion. The convex corners of the second elongate portion 412 are rounded with a radius of 10 mm.

The convex corners of the absorbent pad 410, that is the two corners of the first elongate portion 411 distal to the wings 412,414 and the four corners of the second elongate portion are rounded with a radius of 10 mm.

The notches 440a,b on the second elongate portion 412 have a depth of 10 mm and a width of 50 mm, as such, in the present embodiment, the notches extend the entire distance between the rounded corners of their respective ends of the second elongate portion 412.

The absorbent pad 410 is arranged centrally within the wound dressing 401, although the absorbent pad 410 and the covering layer 430 are both "T-shaped" the width of the border region 433 (as measured perpendicular from the periphery of the absorbent pad 410 to the peripheral edge 431 of the covering layer 430) is not uniform. Minima in the width of the border region 433 are found adjacent to a number of points; at the rounded distal end 411c of the first elongate portion 411 the border region has a width of 25 mm, at the two short edges and second long edge of the second elongate portion 412 the width of the border region is 30 mm.

The covering layer notches 432 extend 90 mm from the peripheral edge 431a of the covering layer 430 perpendicular to the centre line E-E and 50 mm from the two edges parallel 431b to the centre line E-E.

The interior region 420' of the adhesive skin contact layer 420 was a width of approximately 12 mm, this provides a sufficient degree of retention of the absorbent pad 312 without hindering the effectiveness thereof.

Of course, the aforementioned teachings could be applied to dressings of other sizes. For example, the dressing could be uniformly scaled down for use with smaller wound sites, or uniformly scaled up for larger wound sites. Equally, the dressing may be non-uniformly scaled, thereby providing a more elongate dressing or a wider dressing as required to suit a particular wound site whilst still retaining the overall T-shaped outline of the absorbent pad 410. Likewise, the width of the border region could be varied, for example from between 10 mm and 50 mm with a wider border region providing greater adhesion to the wound site and an improved seal for the wound dressing cavity.

In use the wound dressing 401 is applied to the wound site such that the central region 402 is arranged over the wound site and is secured in place using the adhesive skin contact layer 420. In the present example, as in the fourth embodiment, the dressing 401 is applied to a patient with a wound on their heel. The central region 402 is applied over the wound with the distal ends of the second elongate portion 412c,d being folded onto the sides of the foot, the wings 413,414 help the wound dressing 401 conform to this fold and also provide an increased volume of absorbent pad 410 proximate to the wound, this provides improved fluid management at the wound site. The notches 440 further improve the ability of the dressing to adapt to the contours of the wound site, providing a preferential position for the dressing 401 to fold at and reducing the possibility for bulging. This effect is particularly pronounced in a preferred embodiment where the covering layer 430 and adhesive skin contact layer 420 comprises flexible film adhesive constructs (for example a polyureathane film with a silicone adhesive), which are easily cut and respond well to being overlapped. The presence of the covering layer notches 432 improves the ease with which the dressing can be applied, it removes (or reduces the likelihood) the need to cut the covering layer on application, streamlining application. The border region 433 still has a greater width than typical dressings with square or rectangular absorbent pads and covering layers thereby providing an improved seal for application of a pressure differential.

The first elongate region 411 is folded around the heel and extends up the back of the ankle. The shape of the wound dressing 401 lends itself to being folded as described above. When folded the border region 433, in particular the portion outward of the wings 313,314 may bulge, due to the increased amount of covering layer 330 in the portion, however the provision of the covering layer notches 432 reduces or prevents this bulge, in the event the bulge is only reduced by the covering layer notches 432 the HCP can readily cut the covering later 430 to assist in conforming the dressing to the wound site without risk of compromising the seal, indeed the large amount of material lends itself to being overlapped once cut, thereby improving the seal.

Next a source of non-atmospheric pressure (for example, when configured for use in NPWT, a pump) is attached to the wound dressing via an airway 437 attached to the aperture 435. To further improve the seal, sealing strips (not shown) may applied to the perimeter of the dressing 401 to ensure that there are no leaks. The provision of the aperture 435 over the first elongate portion 411 of the absorbent pad 410 is particularly advantageous as it is beneficial to the user that the connection to the non-atmospheric pressure (e.g. airway 437) is not arranged above the wound site. In instances where the port is provided above the wound site an external application of pressure onto the dressing, for example where the user lies on it or rests against it, applies greater pressure at the port due to it raised nature.

In other instances, in particular where the wound is linear, for example as a result of an incision, the wound dressing 401 can be arranged such that the second elongate portion 412 extends along the wound, in such instances the wings again provide an increase in absorbent pad volume proximate to the wound site.

The embodiments are described above by way of example only. Many variations are possible without departing from the scope of protection afforded by the appended claims. In particular, whilst the invention has been described with reference to a T-shaped absorbent pad, it will be understood by those skilled in the art that it could equally apply to other shapes such as L-shaped absorbent pads.

## Claims

1. A pressure gradient wound dressing (1,101,201,301,401), the wound dressing comprising an absorbent pad (10,110,210,310,410) having a shape comprising two elongate portions (11,12,111,112,211,212,311,312,411,412), wherein a short edge (11a,111a,211a,311a,411a) of a first elongate portion (11,111,211,311,411) abuts a long edge (12a,112a,212a,312a,412a) of a second elongate portion (12,112,212,312,412), **characterised in that** the wound dressing further comprises at least one wing, (13,14,113,114,213,214,313,314,413,414) the at least one wing (13,14,113,114,213,214,313,314,413,414) extending between a long edge (11c,11d,111c,111d,211c,211d,311c,311d,411c,411d) of the first elongate portion (11,111,211,311,411) and the long edge (12a,112a,212a,312a,412a) of the second elongate portion (12,112,212,312,412).

2. A pressure gradient wound dressing (1,101,201,301,401) according to claim 1 wherein the short edge (11a,111a,211a,311a,411a) of the first elongate portion abuts the long edge (12a,112a,212a,312a,412a) of the second elongate portion at a midpoint of the second elongate portion (12,112,212,312,412).

3. A pressure gradient wound dressing (1,101,201,301,401) according to claim 1 or 2 wherein the wound dressing comprises two wings, a first wing(13,113,213,313,413) extending from a first long edge(11c,111c,211c,311c,411c) of the first elongate portion to the long edge (12a,112a,212a,312a,412a) of the second elongate portion and a second wing(14,114,214,314,414) extending from a second long edge(11d,111d,211d,311d,411d) of the first elongate portion to the long edge of the second elongate portion (12a,112a,212a,312a,412a).

4. A pressure gradient wound dressing (1,101,201,301,401) according to any preceding claim wherein an end of the first elongate portion distal to the second elongate portion comprises an aperture (35,135,235,335,435) for connecting the wound dressing to a source of non-atmospheric pressure.

5. A pressure gradient wound dressing (101,301,401) according to any preceding claim where in the first elongate portion (111,311,411) is tapered, the width decreasing away from the second elongate portion (112,312,412).

6. A pressure gradient wound dressing (1,101,201,301,401) according to any preceding claim wherein the or each wing
(13,14,113,114,213,214,313,314,413,414) has a triangular shape, and wherein one edge (13a,14a,113a,114a,213a,214a,313a,314a,413a,414a) has a concave arc profile.

7. A pressure gradient wound dressing (1,101,201,301,401) according to claim 6 wherein the concave arc has a radius of curvature of at least 20 mm.

8. A pressure gradient wound dressing (1,101,201,301,401) according to claim 6 wherein the concave arc has a radius of curvature of at least 20% the width of one or both elongate portions (11,12,111,112,211,212,311,312,411,412).

9. A pressure gradient wound dressing (1,101,201,301,401) according to claim 6 wherein the concave arc has a radius of curvature of at least 30% the width of one or both elongate portions (11,12,111,112,211,212,311,312,411,412).

10. A pressure gradient wound dressing (1,101,201,301,401) according to any preceding claim wherein the or each wing(13,14,113,114,213,214,313,314,413,414) has an area of at least 1% of the area of the absorbent pad (10,110,210,310,410).

11. A pressure gradient wound dressing (1,101,201,301,401) according to any preceding claim wherein the or each wing(13,14,113,114,213,214,313,314,413,414) has an area of at least 1.5cm².

12. A pressure gradient wound dressing (201,301,401) according to any preceding claim wherein each short edge of the second elongate portion of the absorbent pad comprises a notch (240,340,440).

13. A pressure gradient wound dressing (201,301,401) according to claim 12 wherein the or each notch (240,340,440) has a curved profile.

14. A pressure gradient wound dressing (1,101,201,301,401) according to any preceding claim wherein the wound dressing is a one-piece dressing.

15. A kit comprising at least one pressure gradient wound dressing (1,301) according to any preceding claim and at least one of; a port, an airway (37,337), a source of non-atmospheric pressure (50,350).

## Patentansprüche

1. Druckgradienten-Wundverband (1, 101, 201, 301, 401), wobei der Wundverband ein Saugkissen (10, 110, 210, 310, 410) aufweisend eine Form umfassend zwei längliche Abschnitte (11, 12, 111, 112, 211, 212, 311, 312, 411, 412) umfasst, wobei eine kurze Kante (11a, 111a, 211a, 311a, 411a) eines ersten länglichen Abschnitts (11, 111, 211, 311, 411) an eine lange Kante (12a, 112a, 212a, 312a, 412a) eines zweiten länglichen Abschnitts (12, 112, 212, 312, 412) stößt, **dadurch gekennzeichnet, dass** der Wundverband ferner mindestens einen Flügel (13, 14, 113, 114, 213, 214, 313, 314, 413, 414) umfasst, wobei sich der mindestens eine Flügel (13, 14, 113, 114, 213, 214, 313, 314, 413, 414) zwischen einer langen Kante (11c, 11d, 111c, 111d, 211c, 211d, 311c, 311d, 411c, 411d) des ersten länglichen Abschnitts (11, 111, 211, 311, 411) und der langen Kante (12a, 112a, 212a, 312a, 412a) des zweiten länglichen Abschnitts (12, 112, 212, 312, 412) erstreckt.

2. Druckgradienten-Wundverband (1, 101, 201, 301, 401) gemäß Anspruch 1, wobei die kurze Kante (11a, 111a, 211a, 311a, 411a) des ersten länglichen Abschnitts an die lange Kante (12a, 112a, 212a, 312a, 412a) des zweiten länglichen Abschnitts an einem Mittelpunkt des zweiten länglichen Abschnitts (12, 112, 212, 312, 412) stößt.

3. Druckgradienten-Wundverband (1, 101, 201, 301, 401) gemäß Anspruch 1 oder 2, wobei der Wundverband zwei Flügel umfasst, wobei sich ein erster Flügel (13, 113, 213, 313, 413) von einer ersten langen Kante (11c, 111c, 211c, 311c, 411c) des ersten länglichen Abschnitts zu der langen Kante (12a, 112a, 212a, 312a, 412a) des zweiten länglichen Abschnitts erstreckt und ein zweiter Flügel (14, 114, 214, 314, 414) sich von einer zweiten langen Kante (11d, 111d, 211d, 311d, 411d) des ersten länglichen Abschnitts zu der langen Kante des zweiten länglichen Abschnitts (12a, 112a, 212a, 312a, 412a) erstreckt.

4. Druckgradienten-Wundverband (1, 101, 201, 301, 401) gemäß einem der vorhergehenden Ansprüche, wobei ein Ende des ersten länglichen Abschnitts distal zu dem zweiten länglichen Abschnitt eine Öffnung (35, 135, 235, 335, 435) zum Verbinden des Wundverbands an eine Nicht-Atmosphärendruckquelle umfasst.

5. Druckgradienten-Wundverband (101, 301, 401) gemäß einem der vorhergehenden Ansprüche, wobei sich der erste längliche Abschnitt (111, 311, 411) verjüngt, wobei die Breite von dem zweiten länglichen Abschnitt (112, 312, 412) weg abnimmt.

6. Druckgradienten-Wundverband (1, 101, 201, 301, 401) gemäß einem der vorhergehenden Ansprüche, wobei der oder jeder Flügel (13, 14, 113, 114, 213, 214, 313, 314, 413, 414) eine dreieckige Form hat, und wobei eine Kante (13a, 14a, 113a, 114a, 213a, 214a, 313a, 314a, 413a, 414a) ein konkaves Bogenprofil hat.

7. Druckgradienten-Wundverband (1, 101, 201, 301, 401) gemäß Anspruch 6, wobei der konkave Bogen einen Krümmungsradius von mindestens 20 mm aufweist.

8. Druckgradienten-Wundverband (1, 101, 201, 301, 401) gemäß Anspruch 6, wobei der konkave Bogen einen Krümmungsradius von mindestens 20 % der Breite eines oder beider länglicher Abschnitte (11, 12, 111, 112, 211, 212, 311, 312, 411, 412) aufweist.

9. Druckgradienten-Wundverband (1, 101, 201, 301, 401) gemäß Anspruch 6, wobei der konkave Bogen einen Krümmungsradius von mindestens 30 % der Breite eines oder beider länglicher Abschnitte (11, 12, 111, 112, 211, 212, 311, 312, 411, 412) aufweist.

10. Druckgradienten-Wundverband (1, 101, 201, 301, 401) gemäß einem der vorhergehenden Ansprüche, wobei der oder jeder Flügel (13, 14, 113, 114, 213, 214, 313, 314, 413, 414) eine Fläche von mindestens 1 % der Fläche des Saugkissens (10, 110, 210, 310, 410) aufweist.

11. Druckgradienten-Wundverband (1, 101, 201, 301, 401) gemäß einem der vorhergehenden Ansprüche, wobei der oder jeder Flügel (13, 14, 113, 114, 213, 214, 313, 314, 413, 414) eine Fläche von mindestens 1,5 cm² aufweist.

12. Druckgradienten-Wundverband (201, 301, 401) gemäß einem der vorhergehenden Ansprüche, wobei jede kurze Kante des zweiten länglichen Abschnitts des Saugkissens eine Einkerbung (240, 340, 440) umfasst.

13. Druckgradienten-Wundverband (201, 301, 401) gemäß Anspruch 12, wobei die oder jede Einkerbung (240, 340, 440) ein gekrümmtes Profil aufweist.

14. Druckgradienten-Wundverband (1, 101, 201, 301, 401) gemäß einem der vorhergehenden Ansprüche, wobei der Wundverband ein einteiliger Verband ist.

15. Kit umfassend mindestens einen Druckgradienten-Wundverband (1, 301) gemäß einem der vorhergehenden Ansprüche und mindestens eines von; einer Anschlussstelle, einem Luftkanal (37, 337), einer Nicht-Atmosphärendruckquelle (50, 350).

## Revendications

1. Un pansement (1, 101, 201, 301, 401) de plaie à gradient de pression, le pansement de plaie comprenant un tampon (10, 110, 210, 310, 410) absorbant ayant une forme comprenant deux parties (11, 12, 111, 112, 211, 212, 311, 312, 411, 412) oblongues, dans lequel un petit côté (11a, 111a, 211a, 311a, 411a) d'une première partie (11, 111, 211, 311, 411) oblongue bute sur un grand côté (12a, 112a, 212a, 312a, 412a) d'une deuxième partie (12, 112, 212, 312, 412) oblongue, **caractérisé en ce que** le pansement de plaie comprend en outre au moins une aile (13, 14, 113, 114, 213, 214, 313, 314, 413, 414), la au moins une aile (13, 14, 113, 114, 213, 214, 313, 314, 413, 414) s'étendant entre un grand côté (11c, 11d, 111c, 111d, 211c, 211d, 311c, 311d, 411c, 411d) de la première partie (11, 111, 211, 311, 411) oblongue et le grand côté (12a, 112a, 212a, 312a, 412a) de la deuxième partie (12, 112, 212, 312, 412) oblongue.

2. Un pansement (1, 101, 201, 301, 401) de plaie à gradient de pression suivant la revendication 1, dans lequel le petit côté (11a, 111a, 211a, 311a, 411a) de la première partie oblongue bute sur le grand côté (12a, 112a, 212a, 312a, 412a) de la deuxième partie oblongue en un point médian de la deuxième partie (12, 112, 212, 312, 412) oblongue.

3. Un pansement (1, 101, 201, 301, 401) de plaie à gradient de pression suivant la revendication 1 ou 2, dans lequel le pansement de plaie comprend deux ailes, une première aile (13, 113, 213, 313, 413) s'étendant du premier grand côté (11c, 111c, 211c, 311c, 411c) de la première partie oblongue au grand côté (12a, 112a, 212a, 312a, 412a) de la deuxième partie oblongue et une deuxième aile (14, 114, 214, 314, 414) s'étendant d'un deuxième grand côté (11d, 111d, 211d, 311d, 411d) de la première partie oblongue au grand côté de la deuxième partie (12a, 112a, 212a, 312a, 412a) oblongue.

4. Un pansement (1, 101, 201, 301, 401) de plaie à gradient de pression suivant l'une quelconque des revendications précédentes, dans lequel une extrémité de la première partie oblongue distale de la deuxième partie oblongue comprend une ouverture (35, 135, 235, 335, 435) pour mettre le pansement de plaie en communication avec une source d'une pression non-atmosphérique.

5. Un pansement (101, 301, 401) de plaie à gradient de pression suivant l'une quelconque des revendications précédentes, dans lequel la première partie (111, 311, 411) oblongue est conique, la largeur diminuant, lorsque l'on s'éloigne de la deuxième partie (112, 312, 412) oblongue.

6. Un pansement (1, 101, 201, 301, 401) de plaie à gradient de pression suivant l'une quelconque des revendications précédentes dans lequel la ou chaque aile (13, 14, 113, 114, 213, 214, 313, 314, 413, 414) a une forme triangulaire, et dans lequel un bord (13a, 14a, 113a, 114a, 213a, 214a, 313a, 314a, 413a, 414a) a un profil en arc concave.

7. Un pansement (1, 101, 201, 301, 401) de plaie à gradient de pression suivant la revendication 6, dans lequel l'arc concave a un rayon de courbure d'au moins 20 mm.

8. Un pansement (1, 101, 201, 301, 401) de plaie à gradient de pression suivant la revendication 6, dans lequel l'arc concave a un rayon de courbure représentant au moins 20 % de la largeur de l'une ou des deux parties (11, 12, 111, 112, 211, 212, 311, 312, 411, 412) oblongues.

9. Un pansement (1, 101, 201, 301, 401) de plaie à gradient de pression suivant la revendication 6, dans lequel l'arc concave a un rayon de courbure représentant au moins 30 % de la largeur de la une ou des deux parties (11, 12, 111, 112, 211, 212, 311, 312, 411, 412) oblongues.

10. Un pansement (1, 101, 201, 301, 401) de plaie à gradient de pression suivant l'une quelconque des revendications précédentes, dans lequel la ou chaque aile (13, 14, 113, 114, 213, 214, 313, 314, 413, 414) a une surface représentant au moins 1 % de la surface du tampon (10, 110, 210, 310, 410) absorbant.

11. Un pansement (1, 101, 201, 301, 401) de plaie à gradient de pression suivant l'une quelconque des revendications précédentes, dans lequel la ou chaque aile (13, 14, 113, 114, 213, 214, 313, 314, 413, 414) a une surface d'au moins 1,5 cm².

12. Un pansement (201, 301, 401) de plaie à gradient de pression suivant l'une quelconque des revendications précédentes, dans lequel chaque petit bord de la deuxième partie oblongue du tampon absorbant comprend une encoche (240, 340, 440).

13. Un pansement (201, 301, 401) de plaie à gradient de pression suivant la revendication 12, dans lequel la ou chaque encoche (240, 340, 440) a un profil incurvé.

14. Un pansement (1, 101, 201, 301, 401) de plaie à gradient de pression suivant l'une quelconque des revendications précédentes, dans lequel le pansement de plaie est un pansement en une pièce.

15. Une trousse comprenant au moins un pansement (1, 301) de plaie à gradient de pression suivant l'une quelconque des revendications précédentes et au moins l'un de ; un orifice, une voie (37, 337) d'air, une source de pression non-atmosphérique (50, 350).
